# EUROPEAN PATENT APPLICATION

(11) **EP 4 116 313 A1**
(43) Date of publication of application: **11.01.2023**
(21) Application number: 21184349.5
(22) Date of filing: 07.07.2021
(51) Int. Cl.: C07H 21/04, C07H 1/00, A61P 35/00

(54) **CAP ANALOG FOR THE 5'-END OF EUKARYOTIC MESSENGER RNAS**

(71) Applicant: Westfälische Wilhelms-Universität Münster, 48149 Münster (DE)
(72) Inventor: KLÖCKER, Nils, 48149 Münster (DE); RENTMEISTER, Andrea, 48161 Münster (DE); SPACEK, Petr, 48268 Greven (DE); WEISSENBÖCK, Florian, 48161 Münster (DE)
(74) Representative: Michalski Hüttermann & Partner Patentanwälte mbB

(57) **Abstract**

The present invention relates to a 5'-cap analog according to formula (I) or salts thereof, wherein the 5'-cap analog comprises a photocleavable group fused via a carbamate moiety to the 5'-cap structure.

## Description

The present invention relates to cap analogs for the 5'-end of eukaryotic messenger RNAs.

Almost all eukaryotic messenger RNAs (mRNAs) are modified at their 5'-ends by a cap that is synthesised via addition of a 7-methylguanosine attached via a 5'-5' triphosphate bridge to the first transcribed nucleotide of the mRNA chain. In some species, including mammals, additional methylations occur, mainly at the 2'-O position of the first or the first and second nucleotides of the transcript, denoted cap 1 and cap 2, respectively, where the not methylated cap then is denoted cap 0. The cap structure plays a pivotal role in mRNA metabolism, and only those mRNAs that carry a cap structure are active in translation, i.e. serve as template for protein synthesis. Since the 5'-cap plays a fundamental role in RNA metabolism, modifying the 5'-cap has emerged as a tool for modulation of protein expression and investigation of biological processes.

Cap analogs with different modifications have been developed. A widely used modified cap is the anti-reverse cap analogs (ARCA), a modified cap analog in which the 3'OH group is replaced with OCH3. Because of this substitution, the RNA polymerase can only initiate transcription with the remaining hydroxyl group thus forcing ARCA incorporation in the forward orientation. As a result, a stimulatory effect on translation is achieved. The socalled second generation ARCAs include various modifications of the 5'-5'-phosphate bridge.

WO 2017/053297 A1 discloses 5'-capped RNAs, and methods and compositions for synthesizing 5'-capped RNAs. The described cap containing initiating oligonucleotide primers have natural or modified Cap 0, Cap 1, Cap 2 or trimethylguanosine-Cap (TMG-Cap) structures. Modified caps and capped mRNAs can be employed for production of proteins in various eukaryotic *in vitro* translations systems and in cultured cells.

Alternative strategies to further allow control of location and duration of translation via caged nucleic acids have been developed. For this, numerous positions on nucleobases have been targeted, involving formal substitution of a hydrogen atom with a photocaging group. A photocontrollable cap (PC cap) usable to control the translation of mRNA in a reversible manner through illumination with light was described (Ogasawara, ACS Chem Biol. 2017, 12, 351-356). While introduction of a phenylazo group into the C8 position of guanosine resulted in a thermally unstable isomer, modification of the C2 amine resulted in a photoisomerisable cap. Via *cis-trans* photoisomerization, the 2-meta-methyl-phenylazo cap (mMe-2PA-cap) in the *trans* form silenced translation whereas the *cis* form provided translated protein. Such photocontrollable caps, however, are an artificial compound in mRNA metabolism and will not yield the native nucleoside.

The N7 position of guanosine and the N1 position of adenosine also were used for chemical modification with a photolabile caging group (Anhäuser et al., Angew. Chem. Int. Ed., 2020, 59, 3161-3165). While aryl ketones such as benzophenone and α-hydroxyalkyl ketone yielded photolabile caging groups if installed at the N7 position of guanosine or the N1 position of adenosine, common photocaging groups derived from the *ortho*-nitrobenzyl moiety were not suitable and did not yield the desired photocleavage to release guanosine.

Therefore, it is an object of the present invention to provide a photocontrollable 5'-cap analog, particularly a photocleavable 5'-cap analogue that allows for a regeneration of the native cap. This object is achieved by a photocleavable 5'-cap analog according to formula (I) as given as follows or salts thereof, wherein the 5'-cap analog comprises a photocleavable group A fused via a carbamate moiety to the 5'-cap structure: 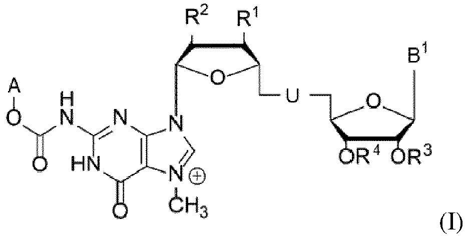 wherein:
- R¹, R²: are independently H, OH or OCH₃;
- R³: is H or CH₃;
- R⁴: is H or an element according to formula (II) 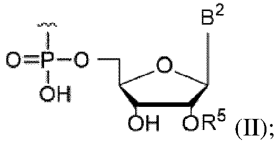 wherein R⁵ is H or CH₃;
- U: is a modified or unmodified oligophosphate or methyleneoligophosphate group;
- B¹, B²: is a modified or unmodified nucleobase; and
- A: is a photocleavable group selected from the group of ortho-nitrobenzyl-based groups, o-nitro-2-phenethyl-based groups, (coumarin-4-yl)methyl-based groups, benzocoumarin-based groups, 2-thionated coumarin-based groups, arylmethyl and arylcarbonylmethyl-based groups, xanthene-based groups, 1,4-benzoquinone-based groups, bimane-based groups, 10H-phenothiazine-based groups, and BODIPY-based groups.

The 5'-cap analog comprising a photocleavable group A that is fused via a carbamate moiety to the N² position of the 7-methylguanosine of the 5'-cap structure allows a photocontrollable regeneration of the native cap. The cap analogs can be enzymatically incorporated at the 5'-end of an mRNA. The 5' cap modification will block translation of a respective mRNA until a radiation-triggered removal. Upon radiation of the 5'-cap analog the photocleavable group is released. This enables a temporal and spatial control of protein biosynthesis. Advantageously, the photocleavable group attached via a carbamate moiety is cleavable providing the native mRNA molecule. Restoration of the native mRNA molecule enables efficient translation. Further, the cleavage results in the generation of carbon dioxide as one of the by-products, which can be metabolised by a cell. The second by-product is formed from the main photocleavable group scaffold. The removal of the photocleavable group thus will release an unmodified capped RNA resulting in uncompromised translation. The 5'-cap analog consequently enables a photocontrollable translation similar to the natural process.

The term "5'-cap" as used herein refers to a cap structure that is attached at the 5'-end to the first transcribed nucleotide of an mRNA chain. The cap comprises a 7-methylguanosine attached via a oligophosphate or methyleneoligophosphate group, such as a 5'-5' triphosphate bridge, to the mRNA.

The term "5'-cap analog" as used herein refers to a functionalized cap structure that can be incorporated into RNA at the 5' end, such as by in vitro transcription, analogous to the natural cap.

The 5'-cap analog can have a cap 0, cap 1 or cap 2 structure. In an embodiment for a cap 0 analog, R³ and R⁵ are hydrogen. In an embodiment for a cap 1 analog, R³ is hydrogen and R⁵ is CH₃. In an embodiment for a cap 2 analog, R³ and R⁵ are CH₃.

The term "photocleavable" refers to a moiety or chemical group that can be removed via irradiation. Such chemical groups also are denoted photoremovable or photosensitive. Such a group can provide a function in regard to the structure it is attached to, such as functioning as hiding or hindering ("caging") a biological activity of the structure it is attached to. In regard to the 5'-cap the photocleavable group hinders the functionality of a natural 5'-cap and the photocleavable group may be related to as a photo-caging group. As used herein, the photocleavable group A may also be referred to as a photocaging group.

In preferred embodiments, A is a photocleavable group selected from the group of ortho-nitrobenzyl-based groups according to formulas (A1) to (A5), o-nitro-2-phenethyl-based groups according to formulas (A6) and (A7), (coumarin-4-yl)methyl-based groups according to formulas (A8) and (A11) to (A14), 2-thionated coumarin-based groups according to formulas (A9) and (A10), benzocoumarin-based groups according to formulas (A15) and (A16), arylmethyl and arylcarbonylmethyl-based groups according to formulas (A17) to (A20), xanthene-based groups according to formula (A21), 1,4-benzoquinone-based groups according to formulas (A22) to (A25), bimane-based groups according to formula (A26), 10H-phenothiazine-based groups according to formula (A27), and BODIPY-based groups according to formulas (A28) to (A34) as given as follows: 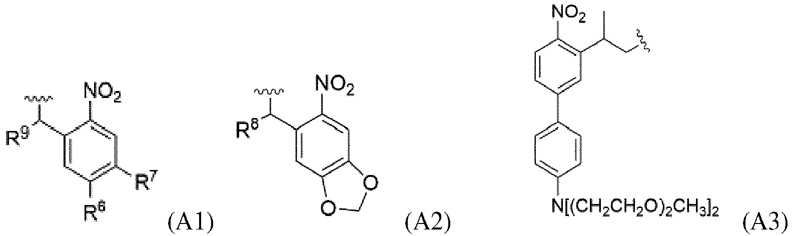 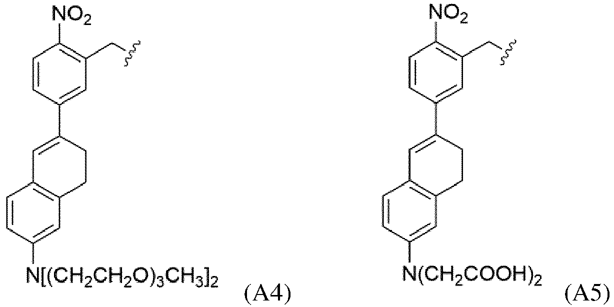 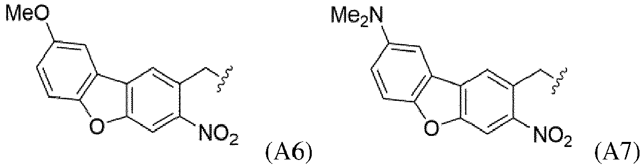 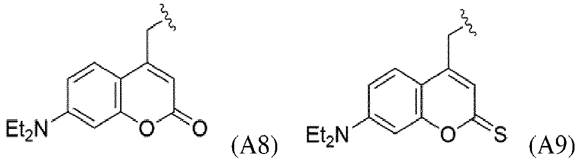 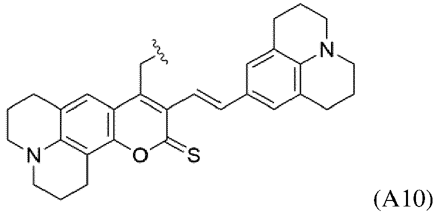 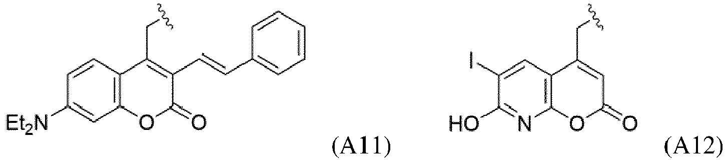 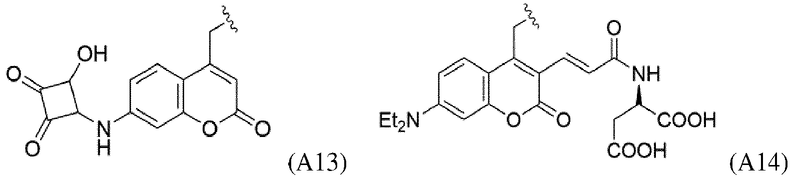 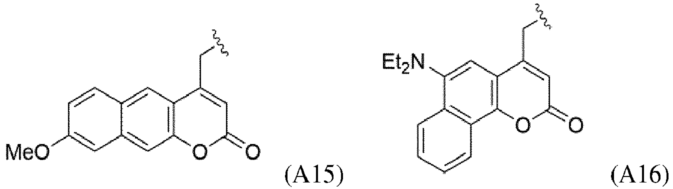 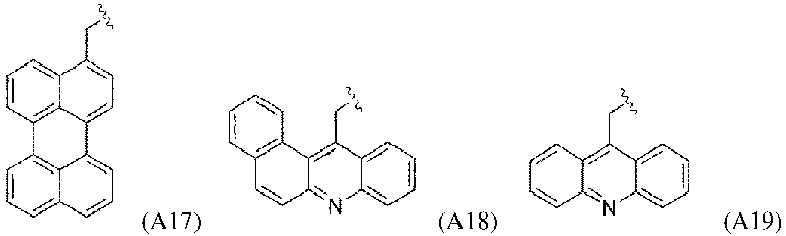 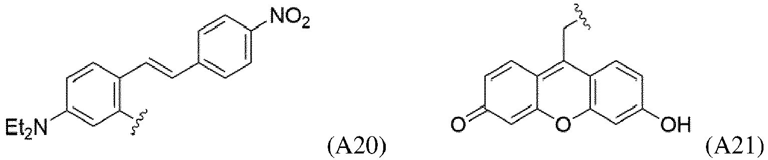 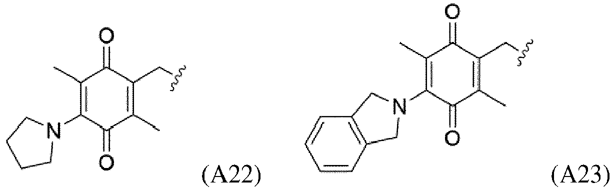 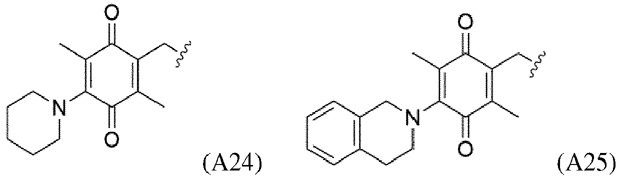 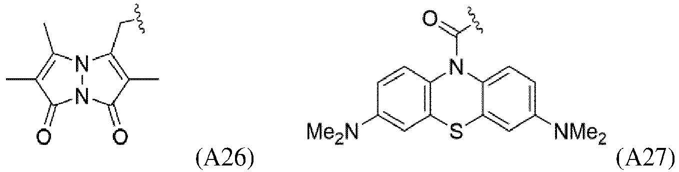 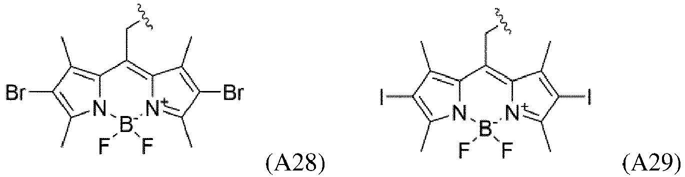 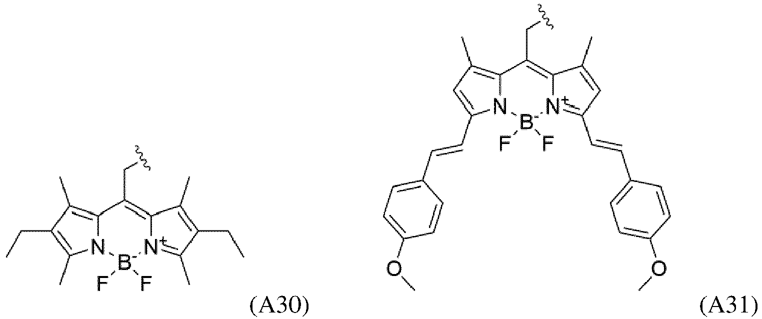 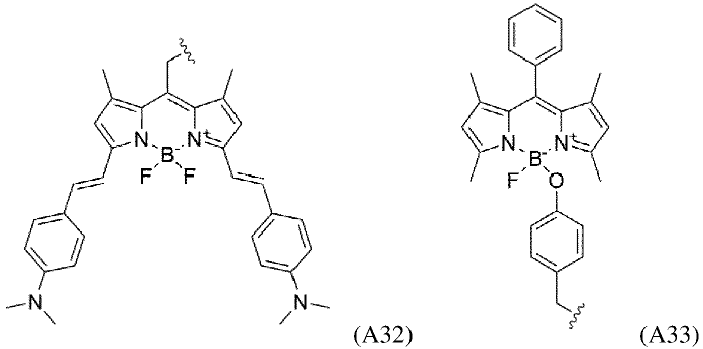 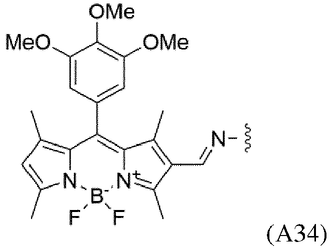 wherein:
R⁶, R⁷ are independently selected from the group of H, OH, NH2, -NH(C₁-C₁₀), -N(C₁-C₁₀)₂, N-methyl piperazine, Br, Cl, C₁-C₁₀ aliphatic alkoxy groups or benzyloxy;
R⁸, R⁹ is independently H or CH₃;
Me is methyl; and
Et is ethyl.

The photocleavable groups show low or tolerable toxicity and thus are usable *in vitro* and *in vivo.*

Preferred photocleavable groups are selected from the group of compounds of the o-nitrobenzyl family according to formulas (A1) and (A2) as given as follows: 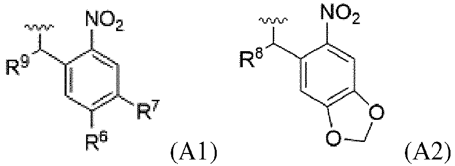 wherein:
- R⁶, R⁷: are independently selected from the group of H, OH, NH₂, -NH(C₁-C₁₀),-N(C₁-C₁₀)₂, N-methyl piperazine, Br, Cl, C₁-C₁₀ aliphatic alkoxy or benzyloxy; and
- R⁸, R⁹: is H or CH₃.

Unless specifically stated otherwise, compounds, groups or substituents denoted with Arabic numerals differ from compounds, groups or substituents denoted with Roman numerals or a combined naming of numerals and letters, that is, compounds, groups or substituents are different compounds, groups or substituents.

The term "C₁-C₁₀ aliphatic alkoxy group" as used herein refers to aliphatic alkoxy group groups having 1 to 10 carbon atoms. Preferred C₁-C₅-alkyl groups may be selected from the group comprising methyl, ethyl, n-propyl, n-butyl and n-pentyl. Preferred linear C₁-C₅-alkyl groups are selected from methyl and ethyl.

Preferably, the aliphatic alkoxy group is a C₁-C₅ aliphatic alkoxy group, particularly a C₁-C₃ aliphatic alkoxy group. In embodiments of the o-nitrobenzyl compound according to formula (A1), R⁶ is selected from the group of H, Cl, OH, NH2, N-methyl piperazine, OCH3 or benzyloxy, R⁷ is hydrogen, and R⁹ is H or CH₃. In other embodiments of the o-nitrobenzyl compound according to formula (A1), R⁶ and R⁷ are hydrogen. In further embodiments of the o-nitrobenzyl compound according to formula (A1), R⁶ and R⁷ are OCH3 and R⁹ is H or CH₃.

In preferred embodiments, A is a photocleavable group selected from the group of ortho-nitrobenzyl compounds according to formulas (A40) to (A44) as given as follows:

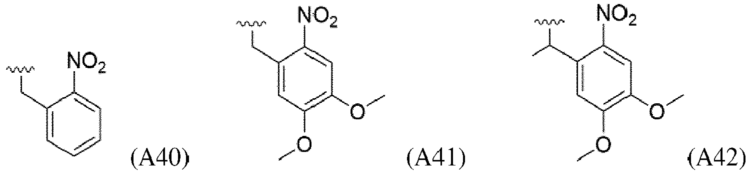

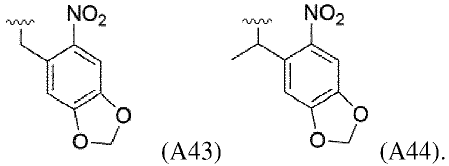

The photocleavable groups according to formulas (A1) and (A2), particularly according to formulas (A40) to (A44), provide advantageous photoremovability upon radiation at 405 nm or 365 nm.

Also the photocleavable groups according to formulas (A3) to (A22) provide good photoremovability upon radiation at wavelengths compatible with a use of the photocleavable 5'-cap analog *in vitro* and *in vivo.* For example, the photocleavable group according to formula (A3) has an absorption profile up to 500 nm or higher, the photocleavable groups according to formulas (A4) and (A5) have an absorption profile up to 550 nm or higher, the photocleavable group according to formula (A6) has an absorption profile up to 450 nm, the photocleavable groups according to formulas (A7) has an absorption profile of about to 424 nm.

The coumarin-based photocleavable groups, comprising the (coumarin-4-yl)methyl compounds, benzocoumarin-based groups and thionated coumarin-based groups according to formulas (A8) to (A16) also provide advantageous photoremovability upon irradiation in a range from 365 nm to 625 nm, for example the photocleavable group according to formulas (A8) at 420 nm, the photocleavable groups according to formula (A9) at 500 nm, the photocleavable group according to formula (A10) has an absorption profile up to 625 nm and absorption maxima at 467 nm, the photocleavable group according to formula (A11) has an absorption maxima at 430 nm, he photocleavable group according to formulas (A12) has an absorption maxima at 365 nm, and the photocleavable group according to formula (A13) has an absorption maxima at 450 nm. The photocleavable group according to formula (A14) is potentially susceptible to two-photon excitation.

The perylene-3-yl-based photocleavable group of formulas (A17) and the (acridin-9-yl)methyl-based according to formulas (A18) and (A19) provide photoremovability upon irradiation at 410 nm or higher, and the xanthene-based photocleavable group according to formula (A21) provides photoremovability upon irradiation at 546 nm. Also the 1,4-benzoquinone-based photocleavable groups according to formulas (A22) to (A25) provide photoremovability. The photocleavable groups according to formulas (A9) and (A21) are preferred for *in vivo* irradiation with green light.

Also the bimane compounds according to formula (A26) and the 10H-phenothiazine based compounds according to formula (A27) provide photoremovability upon irradiation.

The term BODIPY refers to the derivatives of 4,4-difluoro-4-bora-3a,4a-diaza-S-indacene. The BODIPY compounds according to formulas (A28) to (A34) also are preferred photocleavable groups.

The elements B¹ and B² each represent a modified or unmodified nucleobase. As used herein, the term "nucleobase" includes all forms of nucleoside bases found in nature. Base rings most commonly found in naturally occurring nucleosides are purine and pyrimidine rings. Naturally occurring purine rings include, for example, adenine, guanine, and N⁶-methyladenine. Naturally occurring pyrimidine rings include, for example, cytosine, thymine, 5-methylcytosine, pseudouracil. The nucleobases may be modified, such as by substitution with protecting groups and/or halogen such as fluor and include, for example, 2'-deoxy-2'-fluorouridine and 5- fluorouridine. The term modified nucleobase as used herein includes halogen-substituted purines such as 6-fluoropurine, halogen-substituted pyrimidines, N⁶-ethyladenine, N(alkyl)-cytosines or 5-methylcytosine.

Unmodified nucleobases B¹ and B² independently may be selected from guanine, cytosine, thymine, uracil, adenine or N⁶-methyladenine. In embodiments, B¹ and B² independently are a natural nucleobase selected from guanine or adenine. In embodiments, B¹ and B² independently are guanine.

The group U is a modified or unmodified oligophosphate or methyleneoligophosphate group. The group U may be an oligophosphate or methyleneoligophosphate group modified with naturally occurring or not naturally occuring groups such as NH, O, CH₂, S, Se or BH₃. In embodiments, U is a oligophosphate or methyleneoligophosphate group selected from the group of compounds according to formulas (U1) to (U4) as given as follows or salts thereof: 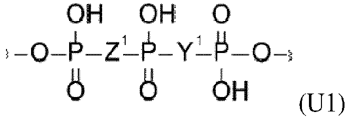 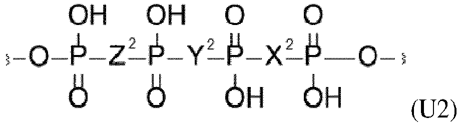 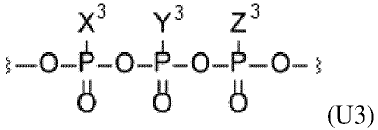 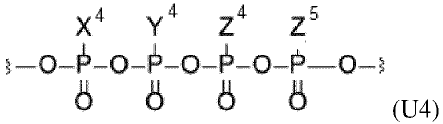 wherein:
- Y¹, Z¹: are independently selected from the group of O, NH and CH₂;
- X², Y², Z²: are independently selected from the group of O, NH and CH₂;
- X³, Y³, Z³: are independently selected from the group of O, S, Se and BH3;
- X⁴, Y⁴, Z⁴, Z⁵: are independently O or S.

The group U may be a di- or triphosphate or a bi- or triphosphonate group modified with one or more sulfur atoms. In embodiments, U is a modified oligophosphate or methyleneoligophosphate group selected from the group of compounds according to formulas (U5) to (U15) as given as follows or salts thereof:

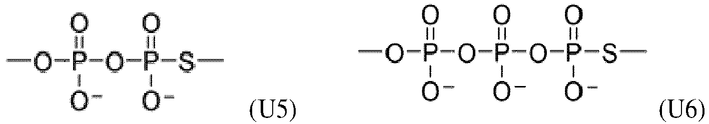

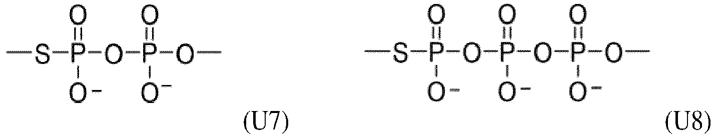

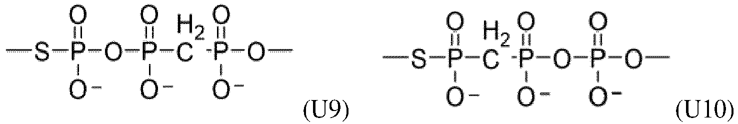

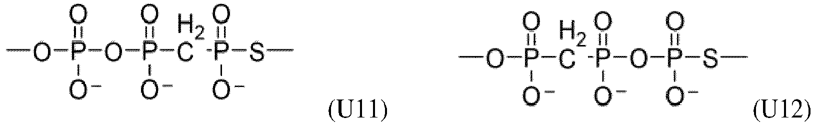

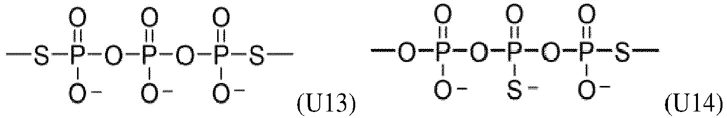

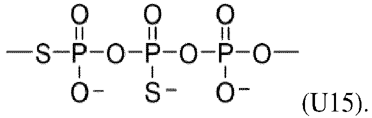

In preferred embodiments, U is a triphosphate or methylenetriphosphate compound according to formula (U1) wherein Y¹ and Z¹ both are O, or one of Y¹ and Z¹ is O and the other CH₂.

The groups R¹, R² independently may be H, OH or OCH3. Preferably, the groups R¹ and R² are OH or OCH3, more preferred the groups R¹ and R² are OH.

The group R³ may be H or CH₃. The photocleavable 5'-cap analog may carry a terminal 3'-OH group that is a valid substrate for RNA polymerase. In other embodiments, the photocleavable 5'-cap analog may carry a terminal 3'-OCH₃ group.

The group R⁴ may be hydrogen or an element according to formula (II) or a salt thereof wherein R⁵ is H or CH₃:

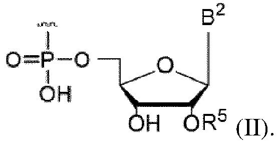

In embodiments R⁴ is an element according to formula (II). The group R⁵ may be hydrogen. In such embodiments, the photocleavable 5'-cap analog may have a structure according to formula (Ia) as given as follows or salts thereof: 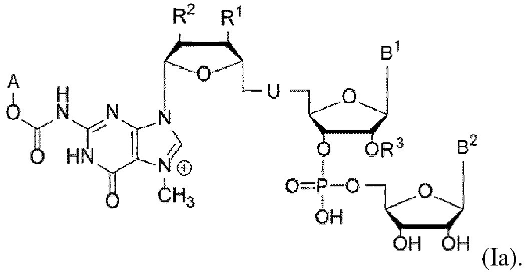 In this embodiment, B¹ may be selected from guanine, cytosine, thymine, uracil, adenine and N⁶-methyladenine, preferably from guanine and adenine. Preferably, B² may be selected from guanine, cytosine, thymine, uracil, adenine or N⁶-methyladenine, preferably from guanine, uracil, and adenine. In preferred embodiments, B¹ is adenine and B² is guanine, or B¹ is adenine and B² is uracil, or B¹ and B² are guanine.

Preferably R⁴ is hydrogen. In such embodiments, the photocleavable 5'-cap analog has a structure according to formula (Ib) as given as follows or salts thereof: 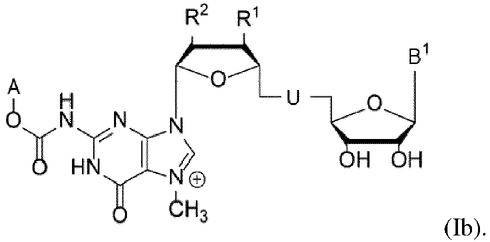 In this embodiment, B¹ may be a natural nucleobase selected from guanine, cytosine, thymine, uracil, adenine and N⁶-methyladenine. Preferably B¹ may be guanine or adenine. Referring to the compounds according to formulas (1a) and (1b), the elements A, B¹, R¹, R², R³ independently may be selected as discussed above.

In embodiments, the photocleavable 5'-cap analog has a structure according to formula (III) as given as follows or salts thereof: 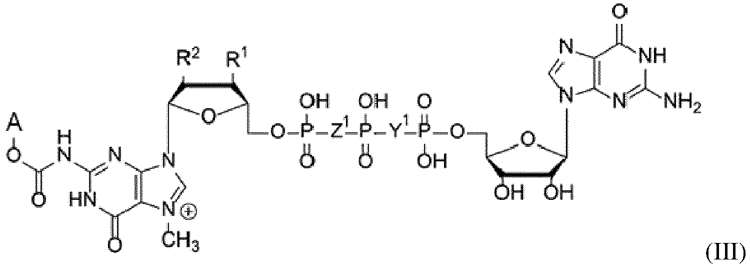 wherein R¹, R² are independently H, OH or OCH₃; and Y¹, Z¹ are independently selected from the group of O, NH and CH₂. Exemplary embodiments of photocleavable 5'-cap analogs according to formula (III) are summarised table 1.

**Table 1: Exemplary embodiments of photocleavable 5'-cap analogs according to formula (III)**

| Abbreviation | R¹ | R² | Y¹ | Z¹ |
|---|---|---|---|---|
| A-OC(O)-m7GpppG | OH | OH | O | O |
| A-OC(O)-m₂^{7,3'-O}GpppG | OCH₃ | OH | O | O |
| A-OC(O)-m₂^{7,2'-O}GpppG | OH | OCH₃ | O | O |
| A-OC(O)-m⁷GppCH₂pG | OH | OH | CH₂ | O |
| A-OC(O)-m⁷GpCH₂ppG | OH | OH | O | CH₂ |
| A-OC(O)-m₂^{7,3'-O}GppCH₂pG | OCH₃ | OH | CH₂ | O |
| A-OC(O)-m₂^{7,3-O}GpCH₂ppG | OCH₃ | OH | O | CH₂ |
| A-OC(O)-m₂^{7,2'-O}GppCH₂pG | OH | OCH₃ | CH₂ | O |
| A-OC(O)-m7GppNHpG | OH | OH | NH | O |
| A-OC(O)-m7GpNHppG | OH | OH | O | NH |
| A-OC(O)-m₂^{7,2'-O}GppNHpG | OH | OCH₃ | NH | O |
| A-OC(O)-m₂^{7,2'-O}GpNHppG | OH | OCH₃ | O | NH |

In other embodiments, the photocleavable 5'-cap analog has a structure according to formula (IV) as given as follows or salts thereof: 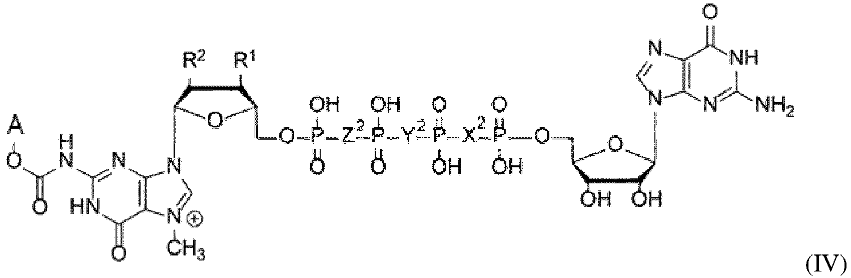 wherein R¹, R² are independently selected from the group of OH or OCH3; and X², Y², Z² are independently selected from the group of O, NH and CH₂. Exemplary embodiments of photocleavable 5'-cap analogs according to formula (IV) are summarised table 2.

**Table 2: Exemplary embodiments of photocleavable 5'-cap analogs according to formula (IV)**

| Abbreviation | R¹ | R² | X² | Y² | Z² |
|---|---|---|---|---|---|
| A-OC(O)-m₂^{7,2'-O}Gp₄G | OH | OCH₃ | O | O | O |
| A-OC(O)-m₂⁷2'dGp₄G | OH | H | O | O | O |
| A-OC(O)-m⁷GpppCH₂pG | OH | OH | CH₂ | O | O |
| A-OC(O)-m⁷GppCH₂ppG | OH | OH | O | CH₂ | O |
| A-OC(O)-m⁷GpCH₂pppG | OH | OH | O | O | CH₂ |
| A-OC(O)-m₂^{7,2'-O}GpppCH₂pG | OH | OCH₃ | CH₂ | O | O |
| A-OC(O)-m₂^{7,2'-O}GppCH₂pG | OH | OCH₃ | O | CH₂ | O |
| A-OC(O)-m₂^{7,2'-O}GpCH₂pppG | OH | OCH₃ | O | O | CH₂ |
| A-OC(O)-m⁷GpppNHpG | OH | OH | NH | O | O |
| A-OC(O)-m₂^{7,2'-O}GpppNHpG | OH | OCH₃ | NH | O | O |
| A-OC(O)-m⁷GpNHpppG | OH | OH | O | O | NH |
| A-OC(O)-m₂^{7,2'-O}GpNHpppG | OH | OCH₃ | O | O | NH |

In other embodiments, the photocleavable 5'-cap analog has a structure according to formula (V) as given as follows or salts thereof: 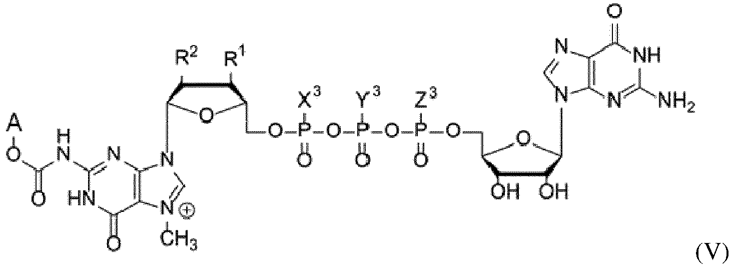 wherein R¹, R² are independently OH or OCH₃; and X³, Y³, Z³ are independently selected from the group of O, S, Se and BH3. Exemplary embodiments of photocleavable 5'-cap analogs according to formula (V) are summarised table 3.

**Table 3: Exemplary embodiments of photocleavable 5'-cap analogs according to formula (V)**

| Abbreviation | R¹ | R² | X³ | Y³ | Z³ |
|---|---|---|---|---|---|
| A-OC(O)-m⁷GpppsG | OH | OH | O | O | S |
| A-OC(O)-m⁷GppspG | OH | OH | O | S | O |
| A-OC(O)-m⁷GpsppG | OH | OH | S | O | O |
| A-OC(O)-m₂^{7,2'-O}GpppsG | OH | OCH₃ | O | O | S |
| A-OC(O)-m₂^{7,2'-O}GppspG | OH | OCH₃ | O | S | O |
| A-OC(O)-m₂^{7,2'-O}GpsppG | OH | OCH₃ | S | O | O |
| A-OC(O)-m₂^{7,2'-O}GppsepG | OH | OCH₃ | O | Se | O |
| A-OC(O)-m⁷Gppp_{BH3}G | OH | OH | O | O | BH₃ |
| A-OC(O)-m⁷Gpp_{BH3}pG | OH | OH | O | BH₃ | O |
| A-OC(O)-m⁷Gp_{BH3}ppG | OH | OH | BH₃ | O | O |
| A-OC(O)-m₂^{7,2'-O}Gppp_{BH3}G | OH | OCH₃ | O | O | BH₃ |
| A-OC(O)-m₂^{7,2'-O}Gpp_{BH3}pG | OH | OCH₃ | O | BH₃ | O |

In further embodiments, the photocleavable 5'-cap analog has a structure according to formula (VI) as given as follows or salts thereof: 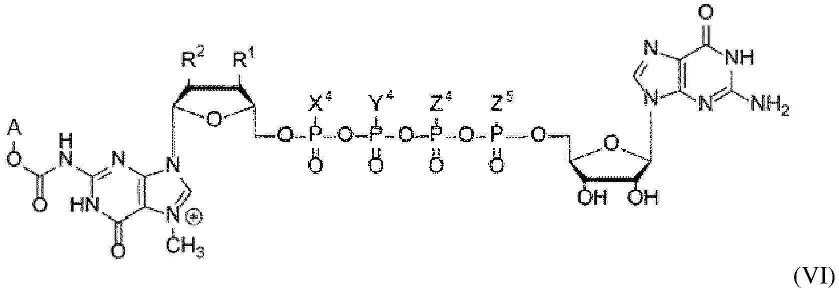 wherein R¹, R² are independently OH or OCH₃ and X⁴, Y⁴, Z⁴, Z⁵ are independently O or S. Exemplary embodiments of photocleavable 5'-cap analogs according to formula (VI) are summarised table 4.

**Table 4: Exemplary embodiments of photocleavable 5'-cap analogs according to formula (VI)**

| Abbreviation | R¹ | R² | X⁴ | Y⁴ | Z⁴ | Z⁵ |
|---|---|---|---|---|---|---|
| A-OC(O)-m₂^{7,2'-O}GppppsG | OH | OCH₃ | O | O | O | S |
| A-OC(O)-m₂^{7,2'-O}GpppspG | OH | OCH₃ | O | O | S | O |
| A-OC(O)-m₂^{7,2'-O}GppsppG | OH | OCH₃ | O | S | O | O |
| A-OC(O)-m₂^{7,2'-O}GpspppG | OH | OCH₃ | S | O | O | O |

In preferred embodiments, the photocleavable 5'-cap analog has a structure according to formula (7) as given as follows or salts thereof:

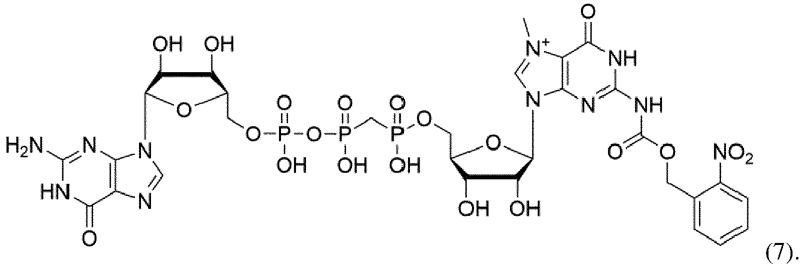

In other preferred embodiments, the photocleavable 5'-cap analog has a structure according to formula (12) as given as follows or salts thereof:

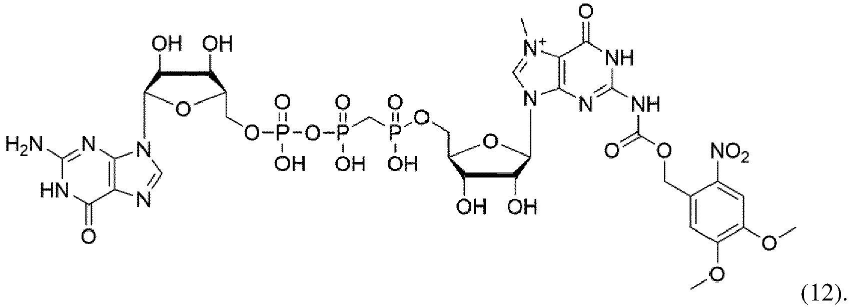

In other preferred embodiments, the photocleavable 5'-cap analog has a structure according to formula (18) as given as follows or salts thereof:

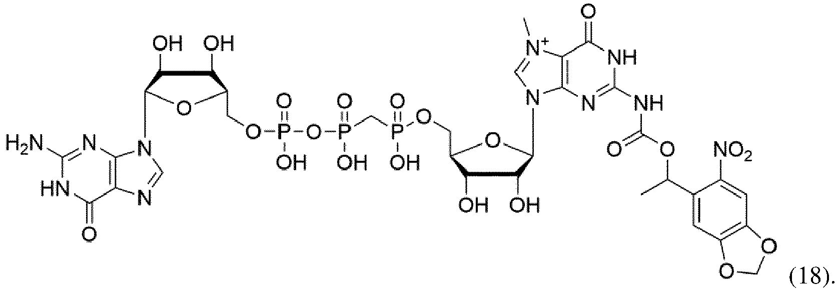

In other preferred embodiments, the photocleavable 5'-cap analog has a structure according to formula (22) as given as follows or salts thereof:

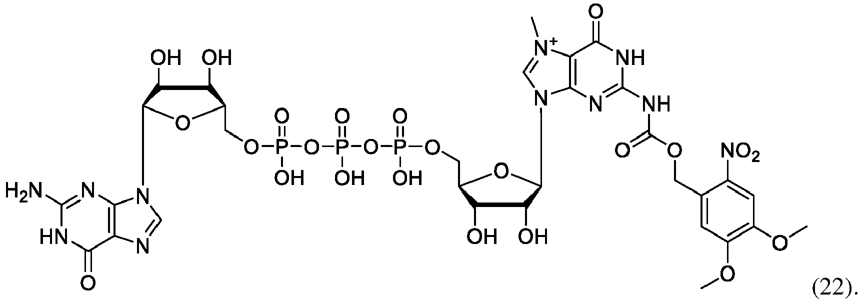

In other preferred embodiments, the photocleavable 5'-cap analog has a structure according to formula (25) as given as follows or salts thereof:

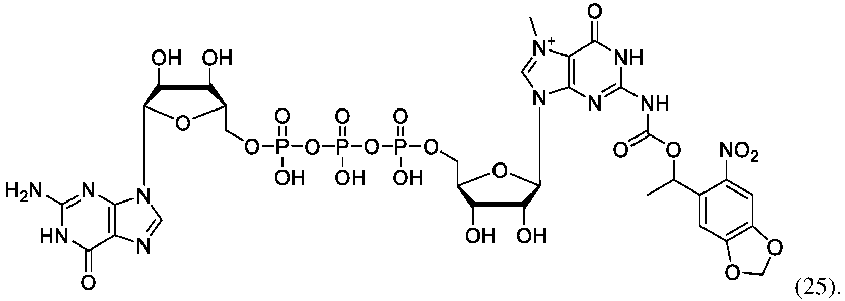

The photocleavable 5'-cap analog comprises oligophosphate or methyleneoligophosphate groups.

The photocleavable 5'-cap analog may be provided in form of its salt. Preferred are potassium and sodium salts. In preferred embodiments, the photocleavable 5'-cap analog is provided in form of a sodium salt.

The photocleavable 5'-cap analog may be used as a tool for studying the function of proteins. Further, the photocleavable 5'-cap analog is usable as therapeutic tool or as a medicament. According to an aspect, the photocleavable 5'-cap analog is usable for inhibiting cap-binding proteins, such as the eukaryotic initiation factor (eIF4E), or cap-degrading enzymes. The inhibition of cap-binding proteins can be annulled via photocleaving of the 5'-cap analog. A cap-binding protein is, for example, eukaryotic translation initiation factor 4 (eIF4E). Examples for cap-degrading enzymes are DcpS and Dcp1-2. Dcp2 is a bilobed enzyme composed of an N-terminal portion, which binds the activator Dcp1 and a catalytic domain, which carries out cap hydrolysis. Decapping Scavenger (DcpS) enzyme is an mRNA decapping pyrophosphatase.

It was suggested that targeting eIF4E is a potentially promising therapeutic strategy to inhibit aberrant pain plasticity. eIF4E integrates signals from two major signaling pathways, ERK and mTORC1, both of which have important functions in the development of pain. Further, eIF4E mainly regulates the translation of a subset of mRNAs involved in cell growth, proliferation, immune responses, and neuronal plasticity.

A further aspect thus relates to a use of a photocleavable 5'-cap analog as described herein for use as a medicament, particularly for use in the treatment of pain or cancer.

A further aspect relates to a pharmaceutical composition comprising a photocleavable 5'-cap analog as described herein and a pharmaceutically acceptable carrier. In embodiments, the pharmaceutical composition is usable in the treatment of pain or cancer.

A further aspect relates to an RNA molecule comprising a photocleavable 5'-cap analog as described herein. For the description of the 5'-cap analog and its elements reference is made to the description above.

RNA molecules are usable as therapeutic tools and an RNA molecule comprising the photocleavable 5'-cap analog is usable as a medicament. Another aspect relates to an RNA molecule comprising a photocleavable 5'-cap analog as described herein for use as a medicament. In preferred embodiments, the RNA molecule comprising a photocleavable 5'-cap analog as described herein is for use as a vaccine or for protein replacement therapy. Further, the RNA molecule comprising a photocleavable 5'-cap analog as described herein may be for use in the treatment of pain or cancer.

A further aspect relates to a pharmaceutical composition comprising an RNA molecule comprising a photocleavable 5'-cap analog as described herein and a pharmaceutically acceptable carrier. In embodiments, the pharmaceutical composition is usable as a vaccine. In embodiments, the pharmaceutical composition is usable for protein replacement therapy. In further embodiments, the pharmaceutical composition may be usable in the treatment of pain or cancer.

The term "pharmaceutical or pharmacologically acceptable" refers to molecular entities and compositions that do not produce an adverse, allergic or other untoward reaction when administered to a subject, such as, for example, a human, as appropriate. The composition may comprise a pharmaceutical carrier, which can be, for example, a solid, liquid, or gas. Suitable carriers preferably are liquid and correspond to the substances ordinarily employed in formulation technology for pharmaceutical formulations. For compositions convenient pharmaceutical media may be employed. For example, water, buffers, glycols, oils, alcohols and the like may be used to form liquid preparations such as solutions. The pharmaceutical composition may be produced under sterile conditions using standard pharmaceutical techniques well known to those skilled in the art.

A further aspect relates to a use of a photocleavable 5'-cap analog as described herein in a method of synthesising an RNA molecule. Using the photocleavable 5'-cap analog various RNA molecules, particularly messenger RNA molecules, which are protected from translation but upon irradiation are activatable, can be synthesised in an easy and straightforward way.

A further aspect relates to a method for synthesising an N-protected adenosine or guanosine, which is particularly usable in a method for synthesising a photocleavable 5'-cap analog comprising a carbamate moiety as described herein.

The method for synthesising adenosine or guanosine fused via a carbamate moiety to an ortho-nitrobenzyl group (N-protected adenosine or guanosine) comprises the steps of:
a) providing a nucleoside selected from adenosine or guanosine;
b) reacting the hydroxyl and carbonyl oxygens of the nucleoside with trimethylsilyl chloride to form a protected nucleoside by trimethylsilyl group;
c) reacting the amino group of adenosine or guanosine with phosgene to an isocyanate group;
d) reacting the isocyanate group with optionally substituted o-nitrobenzyl alcohol or an optionally substituted compound according to formula (6) as follows: 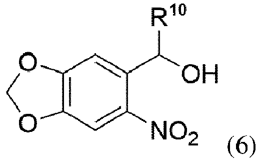 wherein R¹⁰ is selected from H or methyl
   to yield the nucleoside fused to o-nitrobenzyl, 4,5-dimethoxy-2-nitrobenzyl or the optionally substituted compound according to formula (6) via a carbamate moiety; and
e) optionally removing the trimethylsilyl groups.

The method allows for a one-pot reaction for synthesising an N-protected adenosine or guanosine compound. Without wishing to be bound to a specific theory, it is assumed that step c) is based on the formation of isocyanate *in situ* generated via phosgene solution.

Preferably, the method steps are performed under inert gas atmosphere, such as under argon atmosphere. In step a) the guanosine or adenosine may be provided in dichloromethane. 4-dimethylaminopyridine may be added, the mixture may be cooled, such as by an ice-water bath, and pyridine may be added subsequently. For reacting the hydroxyl and carbonyl oxygens of the nucleoside with a trimethylsilyl group according to step b), trimethylsilyl chloride may be added. Preferably, the reaction mixture is stirred for two hours or more. Reacting the amino group of adenosine or guanosine with phosgene in step c) preferably is performed under cooling, for example using an ice-water bath. Phosgene may be added as a solution in toluene. The reaction mixture in step c) may stirred for 30 minutes or more. This allows suspending or dissolving a potentially formed precipitate.

In step d) the o-nitrobenzyl alcohol may be an alcohol of the ortho-nitrobenzyl-based groups according to formulas (A1) to (A5), preferably according to formulas (A40) to (A44) or an optionally substituted compound according to formula (6). The o-nitrobenzyl alcohol or compound according to formula (6) may be selected from 2-nitrobenzyl alcohol, 4,5-dimethoxy-2-nitrobenzyl alcohol or 1-(6-nitrobenzo[*d*][1,3]dioxol-5-yl)ethanol.

Preferably, the o-nitrobenzyl alcohol or compound according to formula (6) may be provided as a solution in tetrahydrofuran. Preferably, after adding the o-nitrobenzyl alcohol or compound according to formula (6) the reaction mixture may be stirred and allowed to warm up to room temperature. Resulting from step d) the nucleoside may be fused via a carbamate moiety to o-nitrobenzyl, 4,5-dimethoxy-2-nitrobenzyl, 1-(6-nitrobenzo[*d*][1,3]dioxol-5-yl)ethyl or 6-nitropiperonyl. The fused nucleoside protected by trimethylsilyl groups may be extracted by a separation of the reaction mixture with chloroform and demineralized water and evaporation. Thus, adenosine or guanosine fused via a carbamate moiety to an o-nitrobenzyl group (N-protected adenosine or guanosine) is received.

For further use in a method for synthesising a photocleavable 5'-cap analog the trimethylsilyl groups are removed in step e). Alternatively, the protected compounds may be stored or used in other reactions without removing the trimethylsilyl groups.

For removing the trimethylsilyl groups tetrahydrofuran and aqueous ammonia may be used. The protected compounds may be dissolved in a solvent such as tetrahydrofuran, aqueous ammonia may be added. The mixture may be stirred.

A further aspect relates to a method for synthesising a photocleavable 5'-cap analog comprising a carbamate moiety as described herein, the method comprising synthesising an adenosine or guanosine fused via a carbamate moiety to an o-nitrobenzyl group comprising the steps a) to e) as described above.

Unless otherwise defined, the technical and scientific terms used herein have the same meaning as commonly understood by one of ordinary skill in the art to which this invention belongs.

The examples that follow serve to illustrate the invention in more detail but do not constitute a limitation thereof.

The figures show:
- Figure 1: An overview of the synthesis of a photocleavable 5'-cap analog according to an embodiment of the invention according to example 1.
- Figure 2: A detailed description of the steps 1.1 and 1.2 of the synthesis of example 1.
- Figure 3: An overview of the synthesis of another photocleavable 5'-cap analog according to an embodiment of the invention according to example 2.
- Figure 4: An overview of the synthesis of another photocleavable 5'-cap analog according to an embodiment of the invention according to example 3.
- Figure 5: An overview of the synthesis of another photocleavable 5'-cap analog according to an embodiment of the invention according to example 4.
- Figure 6: An overview of the synthesis of another photocleavable 5'-cap analog according to an embodiment of the invention according to example 5.
- Figure 7: shows in Figures 7 a) and b), respectively, the effect of irradiation at 405 nm and 365 nm on nitropiperonylmethyl fused via a carbamate moiety to guanosine.
- Figure 8: shows the stability of nitropiperonylmethyl fused via a carbamate moiety to guanosine in different media.
- Figure 9: shows the LC-MS Data (triple quad mass measurement) of the photocleavable 5'-cap analog of example 4 irradiated for 15 seconds with 365 nm (3 W) in Figures 9 a) to d), and non irradiated in Figures 9 e) to h), respectively.
- Figure 10: shows the LC-MS Data (Triple quad mass measurement) of the photocleavable 5'-cap analog of example 5 irradiated for 15 seconds with 365 nm (3 W) in Figures 10 a) to d), and non irradiated in Figures 10 e) to h), respectively.
- Figure 11: shows the results of binding studies of eIF4E and the natural cap analog m⁷GpppG in Figure 11 a), and the cap analogs of examples 4 (DMNB) and 5 (NPOM) in Figures 11 b) and c) respectively.
- Figure 12: shows the results of an PAA-gel electrophoresis of GLuc mRNA capped with the photocleavable 5'-cap analog of example 2 (DMT) in Figure 12a) and capped with the photocleavable 5'-cap analog of example 3 (NPOM) compared to a commercial non-modified cap analog in Figure 12b).
- Figure 13: shows *in vitro* translation as luminescence measurements in reticulocyte lysate after irradiation of FLuc-mRNA capped with the photocleavable 5'-cap analog of example 1 (ONB) and a commercial non-modified cap analog (m⁷GppppG).
- Figure 14: shows *in vitro* translation as luminescence measurements in reticulocyte lysate after irradiation of m⁷GpppG capped FLuc-mRNA caged with the photocleavable 5'-cap analog of formulas (22) and (25) (denoted "DMNB m⁷GpppG" and "NPOM m⁷GpppG").
- Figure 15: shows *in vitro* translation as luminescence measurements in reticulocyte lysate after irradiation of m⁷GpppG capped FLuc-mRNA with modified nucleotides m⁵C and N¹methyl-pseudouridine caged with the photocleavable 5'-cap analog of formulas (22) and (25) (denoted "DMNB m⁷GpppG mod" and "NPOM m⁷GpppG mod").
- Figure 16: shows translation in cells as luminescence measurements after irradiation of GLuc-mRNA capped with the photocleavable 5'-cap analog of example 2 (DMT) and a commercial non-modified cap analog (m⁷GpppG).
- Figure 17: shows translation in cells as luminescence measurement after irradiation of HeLa cells transfected with GLuc-mRNA capped with the photocleavable 5'-cap analog of example 2 (DMT) and a commercial non-modified cap analog (m⁷GpppG) with 365 nm/405 nm for 30 s or 60 s.
- Figure 18: shows translation in cells as luminescence measurement after irradiation of HeLa cells transfected with GLuc-mRNA capped with the photocleavable 5'-cap analog example 3 (NPOM) according to the invention and a commercial non-modified cap analog (m⁷GpppG) with 365 nm/405 nm for 15 s, 25 s or 30 s.
- Figure 19: shows translation in cells as luminescence measurements after irradiation of HeLa and HEK293T cells with DMNB caged m7GpppG capped GLuc-mRNA with either regular nucleotides in HeLa and HEK293 cells in a) and b) or modified nucleotides m5C and N1methyl-pseudouridine in HeLa and HEK293 cells in c) and d).
- Figure 20: shows cell viability after irradiation determined via MTT test.
- Figure 21: shows the translation efficiency determined by luminescence measurement.
- Figure 22: shows the Zebrafish mortality after irradiation.

### Materials:

K-Phosphate Buffer (phosphate (K₂HPO₄/KH₂PO₄) buffer for the analytical HPLC): K₂HPO₄ (17.4 g) and KH₂PO₄(27.2 g) were dissolved in ddH₂O (3 L). The pH was tested (pH = 6.5) by calibrated digital pH-meter and filtrated over sterile 0.22 µm filter under vacuum.

2-Nitrobenzyl alcohol was purchased from Sigma-Aldrich. Guanosine, 4,5-Dimethoxy-2-nitrobenzyl alcohol and 6-nitropiperonal (6-nitro-1,3-benzodioxole-5-carbaldehyde, compound 13) were purchased from FluoroChem. Other chemicals were purchased from Acros, Fluka, Fisher Scientific, FluoroChem or Sigma-Aldrich and used without further purification, unless stated otherwise. Unless stated otherwise, solvents were evaporated on a rotary evaporator at 40 °C/2 kPa.

### Methods

¹H, ¹³C and ³¹P spectrometry:
¹H, ¹³C and ³¹P NMR spectra were measured on Agilent DD2 600 spectrometer (600 MHz, 151 MHz and 243 MHz) and Agilent DD2 500 spectrometer (500 MHz, 126 MHz and 202 MHz). The measurements were performed in DMSO-*d*6 or D₂O and referred to residual solvent signal. Complete assignment (if present) is based on heteronuclear correlation experiments HSQC and H, C-HMBC. Chemical shifts (*δ*) are in ppm and coupling constants (*J*) in Hz. The numbering system for the assignment of NMR signals is given for the majority of the compounds individually.

### Mass spectrometry:

High resolution mass spectra were measured on Orbitrap LTQ XL (Thermo Fisher Scientific) spectrometer using ESI technique and Orbitrap Velos Pro (Thermo Fisher Scientific) spectrometer using ESI technique.

For determination of low-resolution mass spectra, HPLC - triple-quadrupole mass spectrometry system was used. The system consists of Agilent 1260 Infinity II with dual λ absorbance detector (G7114A) HPLC part and Agilent Ultivo mass spectrometer with JetStream ion source (type of ESI). Column which was used: Agilent Poroshell 120 EC-C18 (3.0x150/2.7µm). The polar compounds were tested by LC method (eluent 20 mM NH₄OAc buffer (pH = 6)/CH3CN, gradient 0-30%, column temperature = 20 °C). For other compounds the universal LC method (eluent 20 mM NH₄OAc buffer (pH = 6)/CH3CN, gradient 0-100%, column temperature = 40 °C) was used.

### High performance liquid chromatography (HPLC):

For basic HPLC analyses of compounds containing the polar phosphate or phosphonate functional group, Agilent 1260 Infinity HPLC system with diode-array multi λ absorbance detector (G4212B) were used. Column which was used: Macherey-Nagel EP 125/4 NUCLEODUR C18 Pyramid, 5 µm [REF 760201.40]. The polar compounds were tested by universal LC method (eluent K-Phosphate Buffer (pH = 6.5)/CH3CN, gradient 0-30%, column temperature = 20 °C). This equipment was also used as a semi-preparative HPLC system.

### Flash chromatography:

For flash chromatography, Interchim puriFlash^{®} XS520Plus with multi λ absorbance detector and various types of columns were used: a) 330 g column, 50 µm spherical silica gel, Interchim [PF-50SIHP-F0330]; b) 120 g column, 50 µm spherical silica gel, Interchim [PF-50SIHP-F0120]; c) 40 g column, 25 µm spherical silica gel, Interchim [PF-25SIHC-F0040].

For reversed-phase (RP) flash chromatography and preparative HPLC, Büchi PrepChrom C-700 multi λ absorbance detector and various types of columns were used: a) Teledyne ISCO columns RediSepRf^{®} variation "HP C18 Aq GOLD" in sizes 50 g, 100 g and 150 g (RP flash chromatography); b) Macherey-Nagel VP 250/21 NUCLEODUR C18 Pyramid, 10 µm [REF 762285.210] (preparative HPLC).

### Example 1

### Synthesis of the compound according to formula (7)

### Step 1.1 Synthesis of the 2',3',5'-O-Tri(trimethylsilyl)-N²-(2-nitrobenzyl)oxycarbonyl-guanosine (2)

Guanosine (1) (850 mg, 3 mmol, 1 eq.) and 4-dimethylaminopyridine (40 mg, 0.33 mmol, 0.1 eq.) were suspended in dry DCM (60 mL) in a round bottom Schlenk flask under argon atmosphere. The mixture was cooled by an ice-water bath and dry pyridine (10 mL) was added subsequently. Trimethylsilyl chloride (2.4 mL, 18.9 mmol, 6.3 eq.) was added dropwise and the reaction mixture was stirred for 2 hours. At 0 °C a solution of phosgene in toluene (3 mL of 15wt% solution = roughly 4.2 mmol, 1.4 eq.) was added dropwise. The formation of a yellow precipitate was observed. The reaction mixture was stirred for 30 min to suspend/dissolve the precipitate. Subsequently, the solution of 2-nitrobenzyl alcohol (1.8 g, 11.8 mmol, 3.9 eq.) in dry THF (10 mL) was added and the reaction mixture was stirred overnight to gradually warm up to room temperature. The reaction mixture was transferred to a separation funnel with chloroform (60 mL) and demineralized water (80 mL). The organic layer was extracted by demineralized water (3x 80 mL) and solvent was evaporated *in vacuo.* Crude product (2) was obtained as brown oily solid and used in the next reaction step without purification.

### Step 1.2 Synthesis of N²-(2-nitrobenzyl)oxycarbonylguanosine (3)

The crude guanosine derivative 2 was dissolved in THF (50 mL) and transferred to the tall test tube with cross magnetic stir bar. Solution was vigorously stirred and aqueous ammonia was added (2.5 mL of roughly 25% solution). Reaction mixture was stirred overnight to form white-beige precipitate (compound 3). Solid and suspended precipitate was centrifuged (3220 rcf, 4°C, 10 min). The supernatant has been removed and the precipitate was freezed in liquid nitrogen and freeze-dried to obtain compound 3 as a white-beige solid.

### Step 1.3 Synthesis of N²-(2-nitrobenzyl)oxycarbonylguanosine 5'-methylene(bisphosphonate) (4)

The guanosine derivative 3 (124 mg, 0.27 mmol, 1 eq.) was suspended in dry trimethyl phosphate (TMP) (2 mL) in a Schlenk flask (10 mL) under argon atmosphere. Methylenebis(phosphonic dichloride) (170 mg, 0.68 mmol, 2.5 eq.) was dissolved in dry TMP (3 mL) in another Schlenk flask (10 mL) under argon atmosphere. Both mixtures were cooled by an ice-water bath and stirred until methylenebis(phosphonic dichloride) was fully dissolved. The solution of methylenebis(phosphonic dichloride) in TMP was transferred dropwise into the suspension of compound 3. Reaction mixture was stirred for 2.5 hours and then transferred in several portions to the cooled (ice-water bath) solution of TEAB (20 mL, 0.82 M). The resulting solution (pH ∼ 7, by pH paper) was extracted by MTBE (4x 30 mL) to remove the majority of TMP. The aqueous phase was evaporated *in vacuo* (bath = 35 °C, pressure gradually from 200 mbar to 25 mbar). The residue was dissolved in demineralized water (6 mL) and was purified by flash chromatography (C18-aq 100g; eluent TEAB (0.1M)/CH₃CN, gradient 0-60%) and freeze-dried to obtain 108 mg (yield: 44%) of compound 4 as a fluffy lyophilizate. The obtained form was apparently tris(triethylammonium) salt.

### Step 1.4 Synthesis of N²-(2-nitrobenzyl)oxycarbonyl-7-methylguanosine 5'-methylene(bisphosphonate) (5)

Tris(triethylammonium) salt of guanosine derivative 4 (108 mg, 117 µmol) was dissolved in dry DMSO (785 µL) in a Schlenk flask (10 mL) under argon atmosphere. The iodomethane (26 µL, 418 µmol) was added and the reaction mixture was stirred for 23 hours. Then, the resulting solution was directly injected onto a chromatographic column and purified by flash chromatography (C18-aq 100g; eluent TEAB (0.1M)/CH₃CN, gradient 0-50%). The pure fraction was freeze-dried to obtain 13.9 mg (yield: 11%) of compound 5 as a fluffy lyophilizate. The obtained form was apparently tris(triethylammonium) / iodo salt.

### Step 1.5 Synthesis of Guanosine 5'-monophosphate imidazolide (6)

Guanosine 5'-monophosphate imidazolide (6) was synthesized from commercially available guanosine 5'-monophosphate disodium salt hydrate (J&K) as described by Warminski, M. et al. ("The synthesis of isopropylidene mRNA cap analogs modified with phosphorothioate moiety and their evaluation as promoters of mRNA translation", Bioorganic & medicinal chemistry letters, 2013, 23(13), pp.3753-3758).
Guanosine 5'-monophosphate imidazolide was synthesized by changing guanosine 5'- monophosphate Na+ salt (407mg, 1 mmole) into the TEA salt on Dowex 50WX1 resin and then mixing it with imidazole (340 mg, 5 mmole) and 2,2'-dithiodipyridine (440 mg, 2 mmole) in anhydrous DMF (12.5 mL) and TEA (140 µL) . Triphenylphosphine (524 mg, 2 mmole) was added, and the mixture was stirred at room temperature overnight. The mixture was poured in a flask containing anhydrous sodium perchlorate (500 mg) dissolved in 60 mL of acetone. After cooling at 4°C for 2 h, the precipitate was filtered, washed twice with 15 mL of cold acetone, and dried in a vacuum desiccator over P₄O₁₀.

### Step 1.6 Synthesis of P1-(N²-(2-Nitrobenzyl)oxycarbonyl-7-methylguanosin-5'-yl)-P3-guanosin-5'-yl 1,2-methylenetriphosphate (7)

ZnCl₂ (50 mg, 367 µmol, 43 eq.) was heated (160 °C) *in vacuo* for 5 hours in a Schlenk flask (10 mL) and then overlaid with argon and cooled to room temperature. Subsequently, guanosine 5'-monophosphate derivative 6 (12 mg, 28 µmol, 3.3 eq.) and magnetic stir bar were added. Tris(triethylammonium) salt of guanosine derivative 5 (9 mg, 8.4 µmol, 1 eq.) was dissolved in dry DMF (1 mL) under argon atmosphere and added to the reaction vessel with other components. The resulting mixture was stirred 19 hours at room temperature and then quenched by adding to the mixture of dH₂O (2 mL) and solution of EDTA (1.5 mL, 0.5M, pH = 7.94). The resulting solution was directly injected onto a chromatographic column and purified by flash chromatography (C18-aq 100g; eluent TEAB (0.1M)/CH₃CN, gradient 0-50%). The appropriate fraction was freeze-dried to obtain compound 7 as a white solid lyophilizate. The compound was used for transcription experiments after confirmation via LC-MS and HRMS.

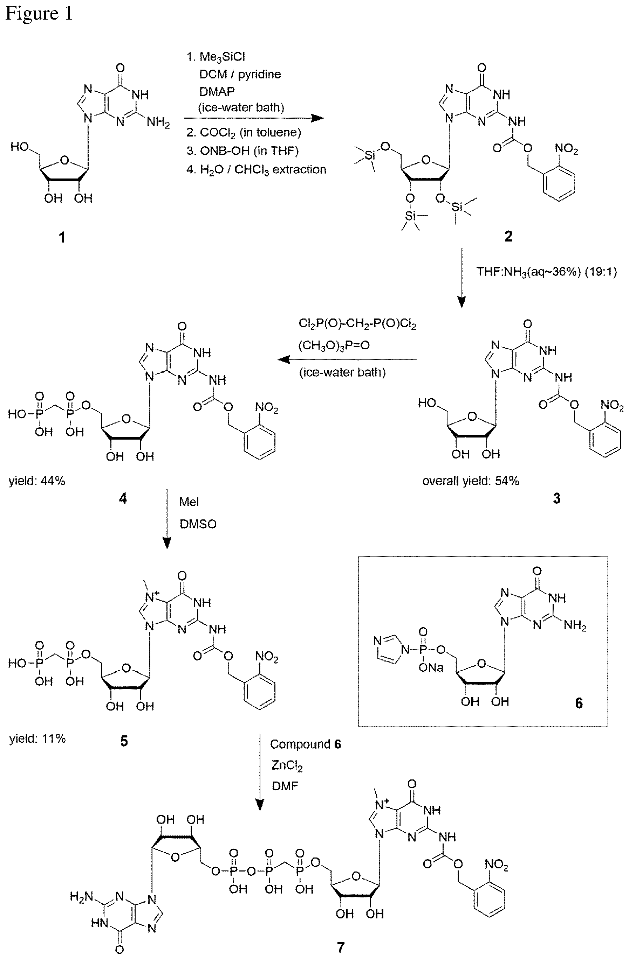

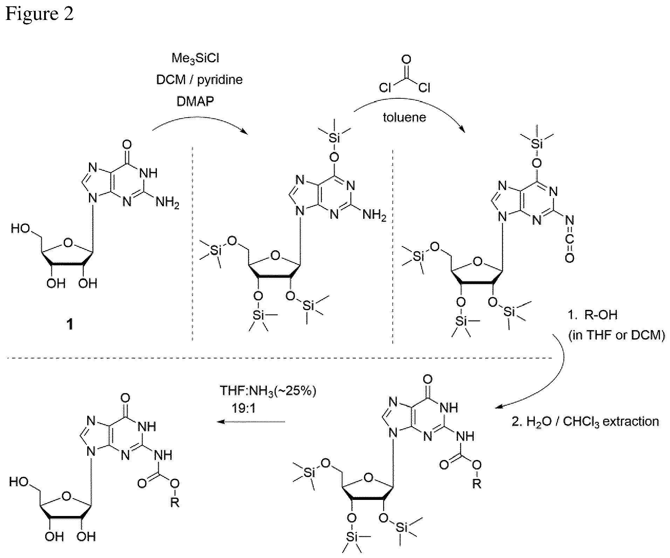

An overview of the synthesis of the photocleavable 5'-cap analog with ortho-nitrobenzyl (ONB) photocleavable group is shown in Figure 1. An overview of the synthesis steps 1.1 and 1.2 is shown in Figure 2, showing a more detailed description for introduction of the photocleavable group at N2 position via a carbamate linker.

### Example 2

### Synthesis of the compound according to formula (12)

### Step 2.1 Synthesis of N²-(4,5-dimethoxy-2-nitrobenzyl)oxycarbonylguanosine (9)

Guanosine (1) (850 mg, 3 mmol, 1 eq.) and 4-dimethylaminopyridine (40 mg, 0.33 mmol, 0.1 eq.) were suspended in dry DCM (60 mL) in a round bottom Schlenk flask under argon atmosphere. The mixture was cooled by an ice-water bath and dry pyridine (10 mL) was added subsequently. Trimethylsilyl chloride (2.4 mL, 18.9 mmol, 6.3 eq.) was added dropwise and the reaction mixture was stirred for 2 hours. At 0 °C a solution of phosgene in toluene (3 mL of 15wt% solution = roughly 4.2 mmol, 1.4 eq.) was added dropwise. The formation of yellow precipitate was observed. The reaction mixture was stirred for 30 min to suspend/dissolve the precipitate. Subsequently, the solution of 4,5-dimethoxy-2-nitrobenzyl alcohol (2.4 g, 11.3 mmol, 3.8 eq.) in dry THF (52 mL) was added and reaction mixture was stirred overnight to gradually warm up to room temperature.

The reaction mixture was transferred to a separation funnel with chloroform (100 mL) and demineralized water (100 mL). The organic layer was extracted by demineralized water (3x 100 mL) and the solvent was evaporated *in vacuo.* Obtained brown oily solid material was dissolved in the THF (50 mL), poured to the reaction vessel (test tube shape) and aqueous ammonia (2.5 mL of 25% solution) was added.

The mixture was stirred vigorously (cross stir bar) overnight to form a white-beige precipitate. Solid and suspended precipitate was centrifuged (3220 rcf, 4°C, 10 min). The supernatant has been removed and the precipitate was frozen in liquid nitrogen and freeze-dried to obtain compound 9 as a white-beige solid.

### Step 2.2 Synthesis of N²-(4,5-dimethoxy-2-nitrobenzyl)oxycarbonylguanosine 5'-methylene(bisphosphonate) (10)

The guanosine derivative 9 (523 mg, 1 mmol, 1 eq.) was suspended in dry trimethyl phosphate (TMP) (12 mL) in a Schlenk flask (50 mL) under argon atmosphere. Methylenebis(phosphonic dichloride) (620 mg, 2.5 mmol, 2.5 eq.) was dissolved in dry TMP (6 mL) in another Schlenk flask (10 mL) under argon atmosphere. Both mixtures were cooled by an ice-water bath and stirred until methylenebis(phosphonic dichloride) was fully dissolved.

The solution of methylenebis(phosphonic dichloride) in TMP was transferred dropwise into the suspension of compound 9. Reaction mixture was stirred for 3 hours and then transferred in several portions (by syringe/needle) to a cooled (ice-water bath) solution of TEAB (20 mL, 1 M).
The resulting solution (pH ∼ 7, by pH paper) was extracted by MTBE (10x60 mL) to remove the majority of TMP. The aqueous phase was evaporated *in vacuo* (bath = 40 °C, pressure gradually from 200 mbar to 15 mbar). The residue was dissolved in TEAB buffer (8 mL ∼ 1M) and was purified by flash chromatography (C18-aq 150g; eluent TEAB (0.1M)/CH₃CN, gradient 0-60%) and freeze-dried to obtain 245 mg (yield: 25%) of compound 10 as a fluffy lyophilizate. The obtained form is apparently tris(triethylammonium) salt.

### Step 2.3 Synthesis of N²-(4,5-dimethoxy-2-nitrobenzyl)oxycarbonyl-7-methylguanosine 5'-methylene(bisphosphonate) (11)

Tris(triethylammonium) salt of compound 10 (114 mg, 116 µmol) was dissolved in dry DMSO (1300 µL) in a Schlenk flask (10 mL) under argon atmosphere. The iodomethane (130 µL, 2 mmol) was added and the reaction mixture was stirred for 7 hours. Then, the resulting solution was mixed with 1 mL of cooled (ice-water bath) TEAB buffer (1 M). After a while, the mixture was injected onto a chromatographic column and purified by flash chromatography (C18-aq 100g; eluent TEAB (0.1M)/CH₃CN, gradient 0-50%). The pure fraction was freeze-dried to obtain 63 mg (yield: 49%) of compound 11 as a fluffy lyophilizate. The obtained form is apparently tris(triethylammonium) / iodo salt [M = 1110.9 g/mol].

### Step 2.4 Synthesis of P1-(N²-(4,5-Dimethoxy-2-nitrobenzyl)oxycarbonyl-7-methylguanosin-5'-yl)-P3-guanosin-5'-yl 1,2-methylenetriphosphate (12)

ZnCl₂ (422 mg, 3.1 mmol, 44 eq.) was heated (160 °C) *in vacuo* for 3 hours in a Schlenk flask (10 mL) and then overlaid with argon and cooled to room temperature. Subsequently, guanosine 5'-monophosphate derivative 6 (101 mg, 235 µmol, 3.3 eq.) and magnetic stir bar were added. Tris(triethylammonium) salt of compound 11 (79 mg, 71 µmol, 1 eq.) was dissolved in dry DMF (3 mL) under argon atmosphere and added to the reaction vessel with other components. The resulting mixture was stirred 67 hours at room temperature and then directly injected onto a chromatographic column and purified by flash chromatography (C18-aq 100g; eluent TEAB (0.1M)/CH₃CN, gradient 0-50%).

The appropriate fraction was freeze-dried, dissolved in the ddH₂O (2 mL) and purified on POROS-50HQ column (eluent ddH₂O:TEAB (1M), gradient 0-100%). The result was freeze-dried to obtain a non perfectly pure compound 12 as a white solid lyophilizate (17.4 mg). The solid was dissolved in solution of TEAB (2 mL, 1M) and purified again, in this case by preparative HPLC (C18 VP 250/21, 10 µm; eluent TEAB (0.1M)/CH₃CN, gradient 0-50%). The appropriate fraction was freeze-dried to obtain 6 mg of solid lyophilizate. The compound 12 in this form apparently contains Et₃NH⁺ as counter ions. Thus, the material was dissolved in ddH₂O (3 mL) and processed over the ion-exchange resin column (10mL of DOWEX 50W X8 / Na⁺ form), the resulting solution was freeze-dried to obtain compound 12 as a solid trisodium salt.

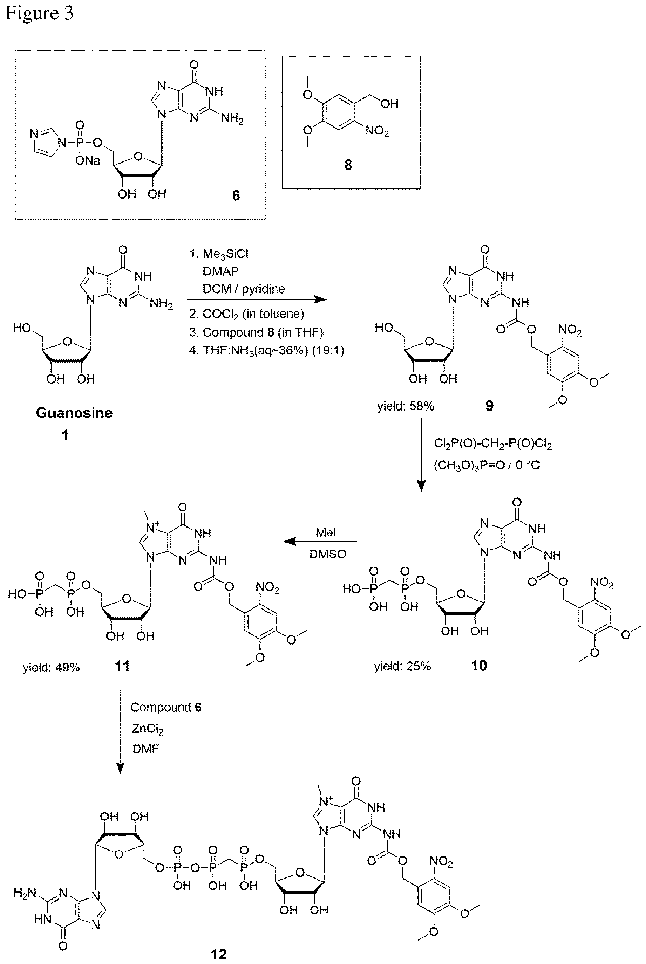

An overview of the synthesis of the photocleavable 5'-cap analog with dimethyoxy-ortho-nitrobenzyl (DMT) photocleavable group is shown in Figure 3.

### Example 3

### Synthesis of the compound according to formula (18)

### Step 3.1 Synthesis of 1-(6-Nitrobenzo[d][1,3]dioxol-5-yl)ethanol (14)

6-Nitropiperonal (13) (5.03 g, 25.8 mmol, 1 eq.) was dissolved in dry DCM (200 mL) under argon in a round-bottom Schlenk flask (500 mL). The mixture was cooled by an ice-water bath for 10 minutes. A solution of Me₃Al (25 mL, 2M in toluene) was added dropwise. The reaction mixture was stirred 1.5 hours and tested by TLC (6:4 / cyclohexane:EtOAc). After full conversion of the starting material, the reaction was quenched carefully with a 1M solution of NaOH (200 mL). The mixture was extracted by DCM (2x 150 mL)/water (1x150 mL). The combined organic phases were washed with dH₂O (2 x 200 mL). The organic solvent was evaporated *in vacuo* and the raw material was purified via flash chromatography (330g SiO₂ column, gradient 80:20 cyclohexane:DCM to 55:40:5 cyclohexane:DCM:MeOH) to obtain the product as an orange-beige solid.

### Step 3.2 Synthesis of N²-(1-(6-nitrobenzo[d][1,3]dioxol-5-yl)ethyl)oxycarbonylguanosine (15)

Guanosine (1) (850 mg, 3 mmol, 1 eq.) and 4-dimethylaminopyridine (40 mg, 0.33 mmol, 0.1 eq.) were suspended in dry DCM (60 mL) in a round bottom Schlenk flask under argon atmosphere. The mixture was cooled by an ice-water bath and dry pyridine (10 mL) was added subsequently. Trimethylsilyl chloride (2.4 mL, 18.9 mmol, 6.3 eq.) was added dropwise and the reaction mixture was stirred for 2 hours. At 0 °C a solution of phosgene in toluene (3 mL of 15wt% solution = roughly 4.2 mmol, 1.4 eq.) was added dropwise. The formation of yellow precipitate was observed. Reaction mixture was stirred for 30 min to suspend/dissolve the precipitate. Subsequently, the solution of 1-(4,5-methylenedioxy-2-nitrophenol) ethan-2-ol 14 (2.04 g, 9.7 mmol, 3.2 eq.) in dry THF (20 mL) was added and reaction mixture was stirred overnight to gradually warm up to room temperature.
The reaction mixture was transferred to a separation funnel with chloroform (100 mL) and demineralized water (100 mL). The organic layer was extracted by demineralized water (3x 100 mL) and the solvent was evaporated *in vacuo.* Obtained brown oily solid material was dissolved in the THF (50 mL), poured to the reaction vessel (test tube shape) and aqueous ammonia (2.5 mL of 25% solution) was added.
The mixture was vigorously stirred (cross stir bar) overnight to form a dark-brown oily precipitate. The liquid was evaporated by introducing nitrogen over a long needle to the liquid (4 hours.). The solid residue was dissolved in DMSO (35 mL) and transferred onto silica gel (25 mL) and mixed. The resulting material was freeze-dried and used as a solid load sample for flash chromatography (column: 25µm-HC silica-gel 40g; eluent CHCl₃/MeOH, gradient 5-30%). The pure fraction was evaporated *in vacuo* to obtain compound 15 as a white-beige solid.

### Step 3.3 Synthesis of N²-(1-(6-nitrobenzo[d][1,3]dioxol-5-yl)ethyl)oxycarbonylguanosine 5'-methylene(bisphosphonate) (16)

Compound 15 (500 mg, 0.96 mmol, 1 eq.) was suspended in dry trimethyl phosphate (TMP) (12 mL) in a Schlenk flask (50 mL) under argon atmosphere. Methylenebis(phosphonic dichloride) (500 mg, 2 mmol, 2.08 eq.) was dissolved in dry TMP (6 mL) in another Schlenk flask (10 mL) under argon atmosphere. Both mixtures were cooled by an ice-water bath and stirred until methylenebis(phosphonic dichloride) was fully dissolved.
The solution of methylenebis(phosphonic dichloride) in TMP was transferred dropwise into the suspension of compound 15. Reaction mixture was stirred 4 hours and then transferred in several portions (by syringe/needle) to a cooled (ice-water bath) solution of TEAB (20 mL, 1 M).
The resulting solution (pH ∼ 7, by pH paper) was extracted by MTBE (10x60 mL) to remove the majority of TMP. The aqueous phase was evaporated *in vacuo* (bath = 40 °C, pressure gradually from 200 mbar to 15 mbar). The residue was freeze-dried overnight. The solid residue was dissolved in TEAB buffer (4 mL ∼ 1 M) (use ultrasonic bath if needed, but it will be probably suspension anyway) and was purified by flash chromatography (C18-aq 100g; eluent TEAB (0.1M)/CH₃CN, gradient 0-60%) and freeze-dried to obtain 129 mg of compound 16 as a fluffy lyophilizate. The obtained form is apparently tris(triethylammonium) salt.

### Step 3.4 Synthesis of N²-(1-(6-nitrobenzo[d][1,3]dioxol-5-yl)ethyl)oxycarbonyl-7-methylguanosine 5'-methylene(bisphosphonate) (17)

Tris(triethylammonium) salt of compound 16 (129 mg, 131 µmol) was dissolved in dry DMSO (1500 µL) in a Schlenk flask (10 mL) under argon atmosphere. The iodomethane (150 µL, 2.3 mmol) was added and the reaction mixture was stirred for 7 hours. Then, the resulting solution was mixed with 1 mL of cooled (ice-water bath) TEAB buffer (2 M). After several minutes, the mixture was injected onto a chromatographic column and purified by flash chromatography (C18-aq 100g; eluent TEAB (0.1M)/CH₃CN, gradient 0-60%). The pure fraction was freeze-dried to obtain 80 mg (yield: 60%) of compound 17 as a fluffy lyophilizate. The obtained form is apparently tris(triethylammonium) / iodo salt [M = 1022.7 g/mol].

### Step 3.5 Synthesis of P1-(N²-(1-(6-nitrobenzo[d] [1,3]dioxol-5-yl)ethyl)oxycarbonyl-7-methylguanosin-5'-yl)-P3-guanosin-5'-yl 1,2-methylenetriphosphate (18)

ZnCl₂ (290 mg, 2.13 mmol, 27 eq.) was heated (170 °C) *in vacuo* for 3 hours in a Schlenk flask (10 mL) and then overlaid with argon and cooled to room temperature. Subsequently, guanosine 5'-monophosphate derivative 6 (120 mg, 276 µmol, 3.5 eq.) and a magnetic stir bar were added. Tris(triethylammonium) salt of compound 17 (80 mg, 78 µmol, 1 eq.) was dissolved in dry DMF (5 mL) under argon atmosphere and added to the reaction vessel with other components. The resulting mixture was stirred 48 hours at room temperature and then directly injected onto a chromatographic column and purified by flash chromatography (C18-aq 100g; eluent TEAB (0.1M)/CH₃CN, gradient 0-50%). The appropriate fraction was freeze-dried, dissolved in ddH₂O (2 mL) and purified on a POROS-50HQ column (eluent ddH₂O:TEAB (1M), gradient 0-100%). The result was freeze-dried to obtain non-perfectly pure compound 18 as a white solid lyophilizate. The product was purified again using the semi-preparative HPLC (37 injections) (C18-Pyramid column - 125x10 mm/5 µm, eluent: 50 mM NH₄OAc buffer (pH = 6) / CH3CN, gradient 5-30%).
The result was freeze-dried to obtain a visible amount (below practical resolution limit of the available laboratory scales) of compound 18 as a white solid lyophilizate.

The solid was dissolved in ddH₂O (2 mL), freeze-dried in the small vial (2.5 mL), dissolved in ddH₂O (500 µL) and freeze-dried again. The result was dissolved in ddH₂O (20 µL) and 0.5 µL of solution was used for HPLC-UV analysis against the standard (ARCA Cap Analog) to roughly estimate the concentration.

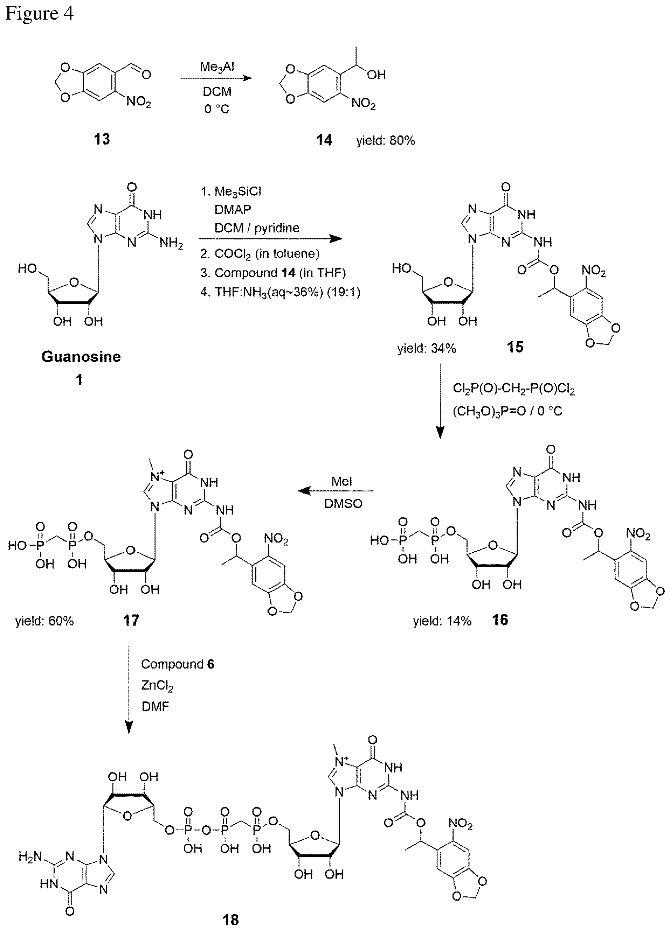

An overview of the synthesis of the photocleavable 5'-cap analog with 1-(6-nitrobenzo[d][1,3]dioxol-5-yl)ethyl (NPOM) photocleavable group is shown in Figure 4.

### Example 4

### Synthesis of the compound according to formula (22)

### Step 4.1 Synthesis of Guanosine 5'-diphosphate imidazolide (19)

Guanosine 5'-diphosphate imidazolide (19) was synthesized from commercially available Guanosine 5'-diphosphate disodium salt hydrate [CAS: 43139-22-6], supplier (Biosynth-Carbosynth, product code: NG08963).
Procedure from reference: General procedure: Guanosine 5'-diphosphate imidazolide was synthesized by changing guanosine 5'-diphosphate Na+ salt (1,11 g, 1.49 mmol, 1 eq.) into the TEA salt on Dowex 50WX8 resin and then mixing it with imidazole (508.6 mg, 7.47 mmol, 5 eq.) and 2,2'-dithiodipyridine (657 mg, 2.98 mmol, 2 eq.) in anhydrous DMF (15 mL) and TEA (207 µL). Triphenylphosphine (782 mg, 2.98 mmol, 2 eq.) was added, and the mixture was stirred at room temperature overnight. The mixture was poured in a flask containing anhydrous sodium perchlorate (1.2 g) dissolved in 60 mL of dry acetone. After cooling at 4 °C for 2 h, the precipitate was filtered, washed twice with 15 mL of cold acetone, and dried in a vacuum desiccator over P₄O₁₀ (yield: 725 mg, 91%).

### Step 4.2 was performed as described in step 2.1 (9)

### Step 4.3 N²-(4,5-Dimethoxy-2-nitrobenzyl)oxycarbonylguanosine 5'-monophosphate (20)

The guanosine derivative 9 (313 mg, 599 µmol, 1 eq.) was suspended in dry trimethyl phosphate (TMP) (5 mL).The solution was cooled to 0 °C and freshly distilled phosphoryl chloride (73 µL, 119 mg, 779 µmol, 1.3 eq.) was added slowly. The reaction mixture was stirred for 1 hour. Subsequently, a precooled solution of TEAB (10 mL, 1 M) was added slowly. The resulting solution (pH ∼ 7, according to pH paper) was extracted with MTBE (10x30 mL) to remove the majority of TMP. The aqueous phase was evaporated *in vacuo.* The residue was dissolved in TEAB buffer (1 mL, 1 M) and purified by flash chromatography (C18-aq 150 g; eluent TEAB (0.1 M)/CH₃CN, gradient 0-50%) and freeze-dried to obtain 217 mg (yield: 45%) of compound 20 as a fluffy lyophilizate as tris(triethylammonium) salt.

### Step 4.4 N²-(4,5-Dimethoxy-2-nitrobenzyl)oxycarbonyl-7-methylguanosine 5'- monophosphate (21)

Tris(triethylammonium) salt of compound 20 (392 mg, 487 µmol, 1 eq.) was dissolved in dry DMSO (3.5 mL) in a Schlenk flask (10 mL) under argon atmosphere. Iodomethane (605 µL, 1.38 g, 9.7 mmol, 20 eq.) was added and the reaction mixture was stirred for 4 hours. Then, the resulting solution was mixed with cooled TEAB (2 mL, 1 M). After five minutes, the mixture was purified by flash chromatography (C18-aq 150 g; eluent TEAB (0.1 M)/CH3CN, gradient 0-50%). Fractions containing the pure product were pooled and freeze-dried to obtain 170 mg (yield: 43%) of compound 21 as a fluffy lyophilizate.

### Step 4.5 P1-(N²-( 4,5- Dimethoxy-2-nitrobenzyl)oxycarbonyl-7-methylguanosin-5'-yl)-P3-guanosin-5'-yl triphosphate (22)

ZnCl₂ (644.7 mg, 4.73 mmol, 43 eq.) was heated (170 °C) *in vacuo* for 3 hours in a Schlenk flask (10 mL) and then overlaid with argon and cooled to room temperature. Subsequently, the guanosine 5'-diphosphate derivative 19 (177.3 mg, 330 µmol, 3 eq.) was added. The tris(triethylammonium) salt of compound 21 (90 mg, 110 µmol, 1 eq.) was dissolved in dry DMF (3 mL) under argon atmosphere and added to the reaction vessel. The resulting mixture was stirred 23 hours at room temperature. Subsequently, ddH₂O (1 mL) and an EDTA solution (1.5 mL, 0.5 M) were added. The reaction mixture was purified by flash chromatography (C18-aq 150 g; eluent TEAB (0.1 M)/CH3CN, gradient 0-50%). The appropriate fractions were pooled, freeze-dried, dissolved in ddH₂O (2 mL) and purified on a POROS-50HQ column (eluent ddH₂O/TEAB (1 M), gradient 0-100%). Fractions containing the pure product were pooled and freeze-dried to obtain 29.6 mg (yield: 24%) of compound 7 as a yellowish solid lyophilizate.

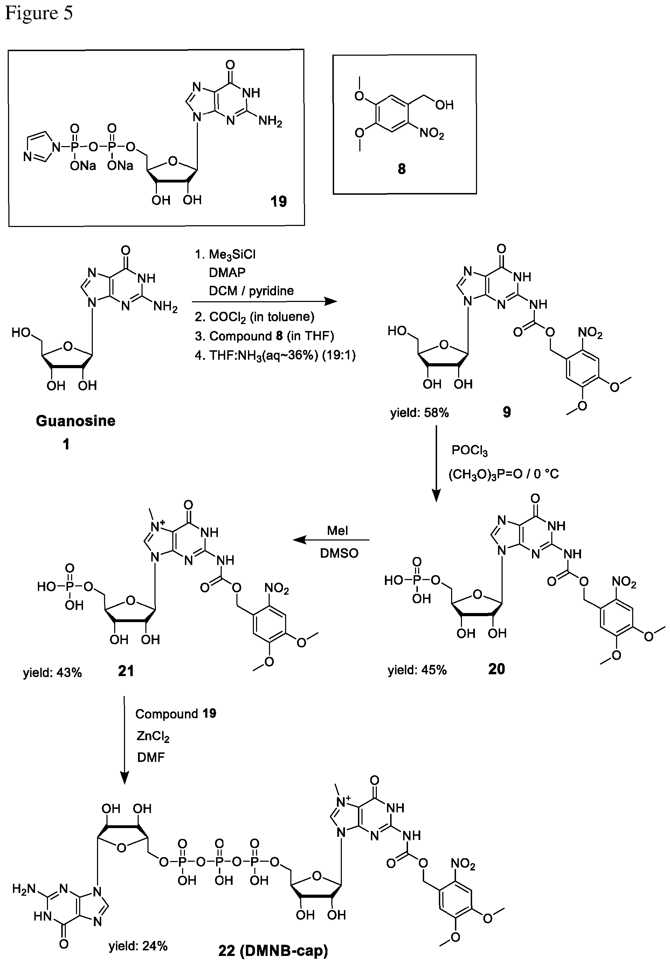

An overview of the synthesis of the compound according to formula (22) is shown in Figure 5.

### Example 5

### Synthesis of the compound according to formula (25)

### Step 5.1 was performed as described in step 3.2 (15)

### Step 5.2 N²-(1-(6-nitrobenzo[d] [1,3]dioxol-5-yl)ethyl)oxycarbonylguanosine 5'- monophosphate (23)

The guanosine derivative 15 (221 mg, 425 µmol, 1 eq.) was suspended in dry trimethyl phosphate (TMP) (4 mL) in a Schlenk flask (20 mL) under argon atmosphere. The solution was cooled to 0 °C and freshly distilled phosphoryl chloride (52 µL, 85 mg, 552 µmol, 1.3 eq.) was added slowly. The reaction mixture was stirred for 1 hour and a precooled solution of TEAB (6 mL, 1 M) was added slowly. The resulting solution (pH ∼ 7, according to pH paper) was extracted with MTBE (10x20 mL) to remove the majority of TMP. The aqueous phase was evaporated in vacuo. The residue was dissolved in TEAB buffer (1 mL, 1 M), purified by flash chromatography (C18-aq 150 g; eluent TEAB (0.1 M)/CH3CN, gradient 0-50%) and freeze-dried to obtain 218 mg (yield: 64%) of compound 23 as tris(triethylammonium) salt.

### Step 5.3 N²-(1-(6-nitrobenzo[d] [1,3]dioxol-5-yl)ethyl)oxycarbonyl-7-methylguanosine 5'- monophosphate (24)

The tris(triethylammonium) salt of compound 23 (166 mg, 207 µmol, 1 eq.) was dissolved in dry DMSO (2 mL) in a Schlenk flask (10 mL) under argon atmosphere. Iodomethane (258 µL, 588 mg 4,14 mmol, 20 eq.) was added and the reaction mixture was stirred for 6 hours. Then, the resulting solution was mixed with cooled TEAB buffer (1.5 mL, 1 M). After five minutes, the mixture was purified by flash chromatography (C18-aq 150 g; eluent TEAB (0.1 M)/CH3CN, gradient 0-50%). The pure fraction was freeze-dried to obtain 68 mg (yield: 40%) of compound 24 as a yellowish lyophilizate.

### Step 5.4 P1-(N²-(1-(6-nitrobenzo[d] [1,3]dioxol-5-yl)ethyl)oxycarbonyl-7-methylguanosin-5'-yl)-P3-guanosin-5'-yl triphosphate (25)

ZnCl₂ (487 mg, 3.57 mmol, 43 eq.) was heated (170 °C) *in vacuo* for 3 hours in a Schlenk flask (10 mL) and then overlaid with argon and cooled to room temperature. Subsequently, guanosine 5'-diphosphate derivative 19 (134 mg, 249 µmol, 3 eq.) was added. The tris(triethylammonium) salt of compound 24 (68 mg, 83 µmol, 1 eq.) was dissolved in dry DMF (2 mL) under argon atmosphere and added to the reaction vessel containing the other components. The resulting mixture was stirred for 32 hours at room temperature. Subsequently, ddH₂O (1 mL) and an EDTA solution (1.5 mL, 0.5 M) were added. The reaction mixture was purified by flash chromatography (C18-aq 150 g; eluent TEAB (0.1 M)/CH₃CN, gradient 0-50%). Fractions containing the product were pooled, freeze-dried, dissolved in ddH₂O (2 mL) and purified on a POROS-50HQ column (eluent ddH₂O/TEAB (1 M), gradient 0-100%). The product containing fractions were pooled and freeze-dried to obtain 43 mg (yield: 39%) of compound 25 as a yellowish solid lyophilizate.

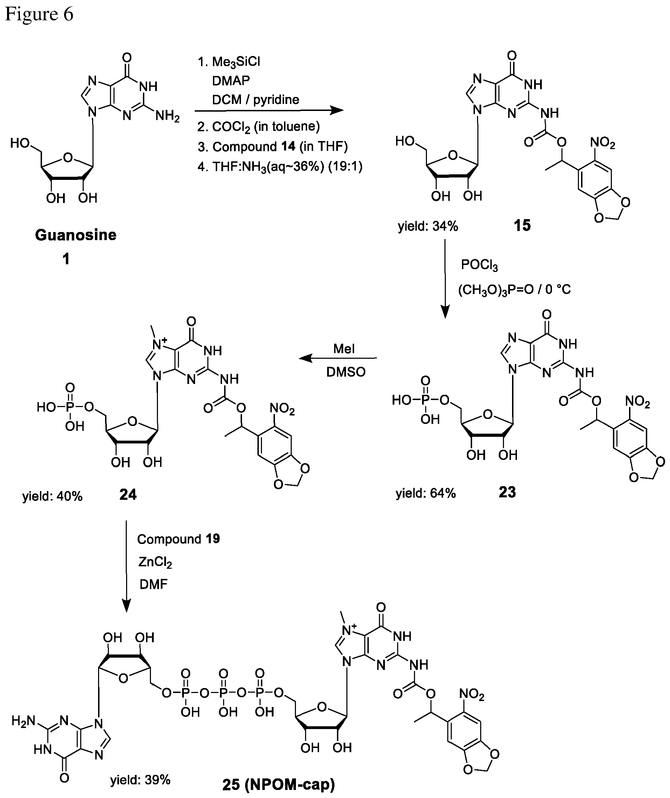

An overview of the synthesis of the photocleavable 5'-cap analog according to formula (25) is shown in Figure 6.

### Biological and biochemical assays:

**Table 5: Devices used during the studies.**

| **Device** | **Manufacturer** |
|---|---|
| Analytical balance Kern ABJ | Kern & Sohn GmbH, Balingen-Frommern, G |
| HPLC Agilent 1260 Infinity | Agilent Technologies Sales & Services GmbH & Co. KG, Waldbronn, G |
| Incubator shaker Multitron Standard | Infors AG, Bottmingen/Basel, CH |
| Imager VersaDocTM MP 4000 | Bio-Rad Laboratories GmbH, München, G |
| Typhoon FLA 9500 laser scanner | GE healthcare, little Chalfont, UK |
| Milli-Q^{®} Reference A+ System | EMD Millipore Corporation, Billerica, USA |
| MyCyclerTM Thermal Cycler | Bio-Rad Laboratories GmbH, München, G |

**Table 6: Synthetic oligonucleotides (purchased from Biolegio).**

| Name | Sequence |
|---|---|
| T7-Promoter-for | TAA TAC GAC TCA CTA TAG GGA AAT AAG (SEQ ID NO: 1) |
| Tail-neu_rev | ATC CAG TCG CGCTGC TCT CGC (SEQ ID NO: 2) |

**Table 7: Enzymes and inhibitors**

| Enzyme | Manufacturer |
|---|---|
| DNase I [1 U/µL] | Thermo Fisher Scientific |
| Phusion^{®} DNA Polymerase [2 U/µL] | Thermo Fisher Scientific |
| Pyrophosphatase [0.1 U/µL] | Thermo Fisher Scientific |
| RiboLock RNase Inhibitor [20 U/µL] | Thermo Fisher Scientific |
| T7-RNA-Polymerase [20 U/µL] | Thermo Fisher Scientific |
| XRN1 [1 U/µL] | New England Biolabs GmbH |
| 5'-Polyphosphatase [20 U/µL] | Biozym Scientific GmbH |

**Table 8: Buffers and solvents used during the studies.**

| Name | Composition |
|---|---|
| APS stock solution | 10 % (*w*/*v*) APS in ddH₂O |
| 6x DNA-Loading Dye | 10 mM Tris; 60 % (*v*/*v*) Glycerol; 60 mM EDTA; 0.03 % (*w*/*v*) bromphenolblue and xylencyanol FF; pH 7.6 |
| HF-buffer (5x) | Composition classified |
| RNA-Loading Dye (2x) | 95 % (*v*/*v*) Formamide, 87 µM SDS, 0.5 mM EDTA |
| TAE-buffer (50x) | 2 M Tris; 50 mM EDTA; pH 8.5 |
| TBE-buffer (5x) | 0.45 M Tris; 0.45 M boric acid; 0.01 M EDTA |
| Transcription buffer (5x) | 200 mM Tris; pH 7.9; 30 mM MgCl₂; 50 mM DTT; 50 mM NaCl; 10 mM spermidine |
| MEM | 1 % glutamine; 1 % 100x non-essential amino acids; 1x fetal calf serum |
| PBS (10x) | 1.4 M NaCl; 0.027 M KCl; 0.1 M Na₂HPO₄; 0.02 M KH₂PO₄ |
| LB-medium | 10 g tryptone, 5 g yeast extract, 10 g NaCl, 900 mL ddH₂O |

**Table 9: Cap-analogs**

| Name | Manufacturer |
|---|---|
| ApppG | Jena Bioscience GmbH |
| m⁷GpppG | Jena Bioscience GmbH |

### Lightsources - LEDs:

UV-A-LEDs (3 W, *λmax* = 365 nm) and blue LEDs (3 W, *λmax* = 405/420/450 nm) were used for the irradiation of cap analogs, caged guanosines, modified mRNA and cells. The spectra were measured with a Jasco FP-8300 fluorescence spectrometer.
The UV-A-LEDs were bought from LED Engine and installed in a selfmade LED-box. The temperature of the box was constantly at 23 °C.

### Agarose gel electrophoresis:

To produce a 1 %-agarose gel, agarose (0.5 g) was added to TAE-buffer (1x) (50 mL). The mixture was heated resulting in a fully dissolved solution and subsequently poured into a gel box. For the analysis, the DNA-fragments were mixed with 2x DNA loading buffer and loaded onto the agarose gel. The electrophoretic separation of the DNA was performed with a voltage of 110 V for 60 min. Visualization was achieved by staining with ethidium bromide and scanning on a Typhoon FLA 9500 laser scanner (GE Healthcare).

### Estimation of conversion rates after irradiation:

Conversions from caged compounds to uncaged compounds were estimated by integrating the peaks of a UV-spectrum that resulted from the HPLC analysis (Agilent infinity 1260).

### Irradiation of protected samples:

When irradiating isolated N2-modified compounds, a final volume of 10 µL in double distilled water was irradiated using a LED (λmax = 365 nm/405 nm/420nm) and then analyzed by RP-HPLC.

### In vitro transcription:

First, the DNA sequence coding for eGFP, Firefly luciferase (FLuc) or Gaussia luciferase (GLuc) mRNAs were amplified from pmRNA-vectors containing the respective sequence. After purification (NucleoSpin Gel and PCR Clean-up, Macherey-Nagel), the resulting linear dsDNA was used as template (200 ng) for *in vitro* T7 transcription performed in transcription buffer (40 mM Tris/HCl, 25 mM NaCl, 8 mM MgCl₂, 2 mM spermidine(HCl)₃) by adding either a A/C/UTP (0.5 mM) mix or a mix with modified nucleotides (A/m⁵C/N¹-methylpseudo-UTP) (0.5 mM), GTP (0.25 mM), GpppG, m7GpppG, ApppG, DMT or NPOM cap analog (1 mM), T7 RNA polymerase (50 U) (Thermo Scientific) and pyrophosphatase (0.1 U) (Thermo Scientific) for 4 h at 37°C. Remaining DNA template was digested in presence of 2 U DNase I for 1 h at 37°C and then mRNAs were purified using the RNA Clean & Concentrator^{™}-5 Kit (Zymo Research). To digest non-capped RNAs, 10 U of the RNA 5'-polyphosphatase (Epicentre) as well as the supplied reaction buffer were added to purified mRNAs. After an incubation period of 30 min at 37 °C, 0.5 U of the 5' - 3' exoribonuclease XRN1 (NEB) and MgCl₂ (5 mM) were added. The reaction mixture was incubated for 60 min at 37 °C. Subsequently, capped mRNAs were purified using the RNA Clean & Concentrator^{™}-5 Kit (Zymo Research).

### In vitro translation and Luminescence measurement:

*In vitro* translation was performed using a eukaryotic cell free protein expression kit from Reticulocyte Lysate (Invitrogen). Experiments were performed in 12.5 µL total volume containing 20 ng of the corresponding FLuc-mRNAs, 50 µM L-methionine and 150 mM potassium acetate. Samples were mixed with 8.5 µL of the reticulocyte lysate and incubated for 90 min at 30°C. Afterwards, 2 µL of the respective translation mix were used in subsequent luminescence measurement. The translation efficiency of the reporter mRNAs was assessed in a luciferase assay using Beetle-juice Luciferase Assay Firefly (*pjk*). Luciferase activity was determined by adding 50 µL freshly prepared substrate solution to the translation mixture. Luminescence was monitored using a Tecan infinite^{®} m1000pro microplate reader with an acquisition time of 3 s (duration of signal acquisition).

### Translation and irradiation in HeLa cells:

HeLa cells were transferred to a 96 well plate 20 h prior transfection. 30.000 cells/well were cultivated in growth Media (MEM, 10 % FCS, 1 % glutamine, 1 % Penicillin, 1 % Streptomycine, 1% non-essential aminoacids). For transfection the growth media was exchanged for MEM-media with 0.6 µL Metafectene^{®} Pro (0.3 µL) and 100 ng mRNA was added per well. 6 h after transfection of the cells the media was exchange by MEM-media, followed by irradiation and another MEM-media exchange. After 24 h post transfection the supernatant in the wells was used for further luminescence measurements. Values of the ApppG capped mRNA represent cap independent translation and were therefore subtracted from the other values.

### Cell viability assay:

3x10⁴ cells in HeLa cell media (100 µL) were transferred in one well of a 96 well-plate. After 24 h the cells were transfected with Metafectene^{®} Pro (0.3 µL) in MEM Earle's media (4.7 µL) and modified mRNA (0.1 µg) in MEM Earle's media (5 µL) for 6 h at 37 °C in a total volume of 100 µL. For subsequent toxicity assays, the media containing the transfection reagent was removed and cells were incubated overnight in fresh serum-free HeLa cell media. Cellular lethality was checked using the LDH Cytotoxicity Detection Kit, cellular viability was checked using the Vybrant^{®} MTT Cell Proliferation Assay Kit (A volume of 100 µL 0.04 M HCl in 2-propanol was used instead of SDS-HCl solution to dissolve the formazan.) and cellular proliferation was checked using the BrdU Cell Proliferation Assay Kit. In each case, 24 h after transfection, absorbance was measured photometrically using the Tecan Infinite^{®} M1000 Pro according to the manufacturer's instructions.

### RP-HPLC analysis:

The enzymatic cap modifications were analyzed and the modified cap analogs were purified by RP-HPLC using a NUCLEODUR^{©} C18 pyramid-column (5 µm, 125 x 10 mm, 4 mm ID) with a flow of 1 mL/min. A linear gradient of ammonium acetate (50 mM, pH 6) and ammonium acetate (100 mM, pH 6) with acetonitrile (1:1) was used for elution.

### PCR for DNA template production:

To amplify the necessary DNA in order to synthesize mRNAs, the linearized vector was added to a solution of ddH₂O, primer, dNTPs, HF-buffer and Phusion (Table 10). The temperature program is shown in Table 11. The annealing temperature was chosen depending on the melting point of the oligonucleotides. The elongation time depended on the length of the DNA-fragment, which ought to be amplified. The PCR product was purified using the NucleoSpin^{®} Gel and PCR Clean-up kit (*Macherey-Nagel*)*.* The concentration was measured with the Tecan Infinite^{©} M1000 PRO.

**Table 10: Composition of the PCR-mix, used for the amplification of DNA-templates**

| Reactant | Final concentration |
|---|---|
| dNTP-Mix [25 mM] | 0.2 mM |
| Phusion | 1 U |
| HF-buffer (5x) | 1x |
| fwd primer [10 µM] | 0.5 mM |
| rev primer [10 µM] | 0.5 mM |
| plasmid | 70 ng |
| ddH₂O | *Ad* 50 µL |

**Table 11: Temperature program of the PCR**

| Phase | Temperature | Time | Cycles |
|---|---|---|---|
| Primary denaturation | 98 °C | 30 s | 1 |
| Denaturation | 98 °C | 10 s | |
| Annealing | 58.9 °C | 30 s | 28 |
| Elongation | 72 °C | variable | |
| Final elongation | 72 °C | 600 s | 1 |

### LC-QQQ analysis:

For determination of mass spectra, HPLC - triple-quadrupole mass spectrometry system was used. The system consists of Agilent 1260 Infinity II with dual λ absorbance detector (G7114A) HPLC part and Agilent Ultivo mass spectrometer with JetStream ion source (type of ESI). Column which was used: Agilent Poroshell 120 EC-C18 (3.0x150/2.7µm). The polar compounds were tested by LC method (eluent 20 mM NH₄OAc buffer (pH = 6)/CH3CN, gradient 0-30%, column temperature =20°C).

### Example 6

### Determination of the effect of irradiation on the photocleavable groups of formulas (A43) and (A44) in vitro

A 500 µM solution of the compound 15, nitropiperonylmethyl fused via a carbamate moiety to guanosine (denoted "NPOM guanosine" or "caged guanosine"), in water/acetone/acetonitrile was irradiated in a 10 µL volume with either 405 nm for 20 s, 40 s or 60 s or with 365 nm for 5 s, 10 s or 20 s, respectively, using a LED power (input 3W and output 147 mW/cm²). HPLC analysis was performed as described above.

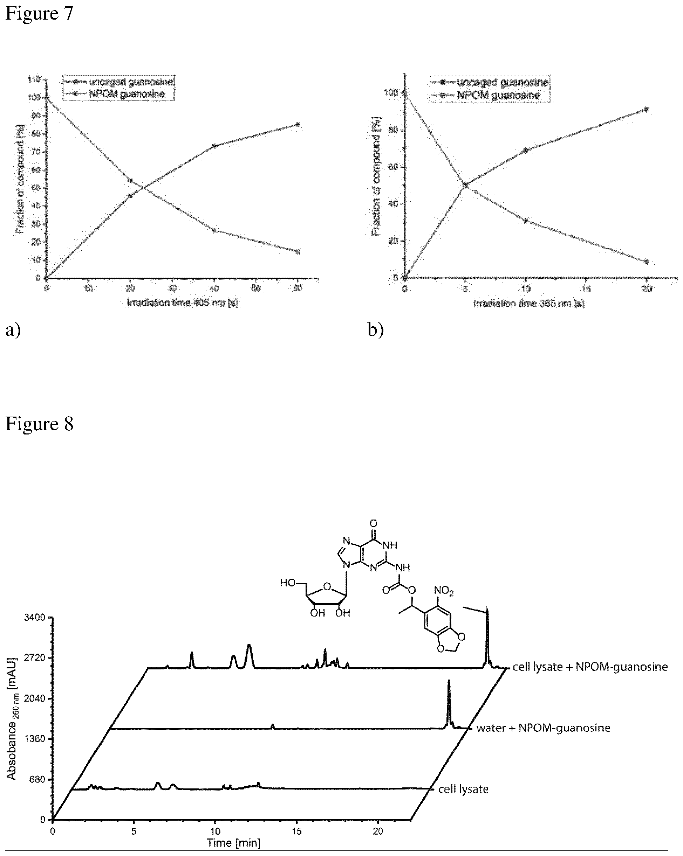

The Figure 7 shows the conversion, based on HPLC analysis, of the compound 15 "NPOM guanosine" to guanosine "uncaged guanosine" upon irradiation at 405 nm and 365 nm in Figures 7 a) and b), respectively. This shows the photocleavable group attached via the carbamate moiety is cleaved by irradiation and that guanosine is regained without side products.

### Example 7

### Determination of the stability of the carbamate linkage and photocleavable groups in vitro

To investigate the stability of the carbamate linker in cellular environment the compound 15, nitropiperonylmethyl fused via a carbamate moiety to guanosine, was studied in water and cell lysate. The cell lysate was produced by ultrasonication of HeLa cells (5*10⁶ cells) in 400 µL 1x PBS buffer. The compound 15, NPOM-guanosine, suspended in water or cell lysate after 24 h incubation at 37 °C was analysed by HPLC-analysis as described above.

The Figure 8 shows the results for compound 15, NPOM-guanosine, suspended in water and cell lysate and the cell lysate. As can be taken from the Figure 8, the compound 15 was stable under these conditions. This indicated that the photocleavable 5'-cap analog is stable under biological conditions.

### Example 8

### Determination of the effect of irradiation on the photocleavable 5'-cap analogs of formula (18) and formula (22)

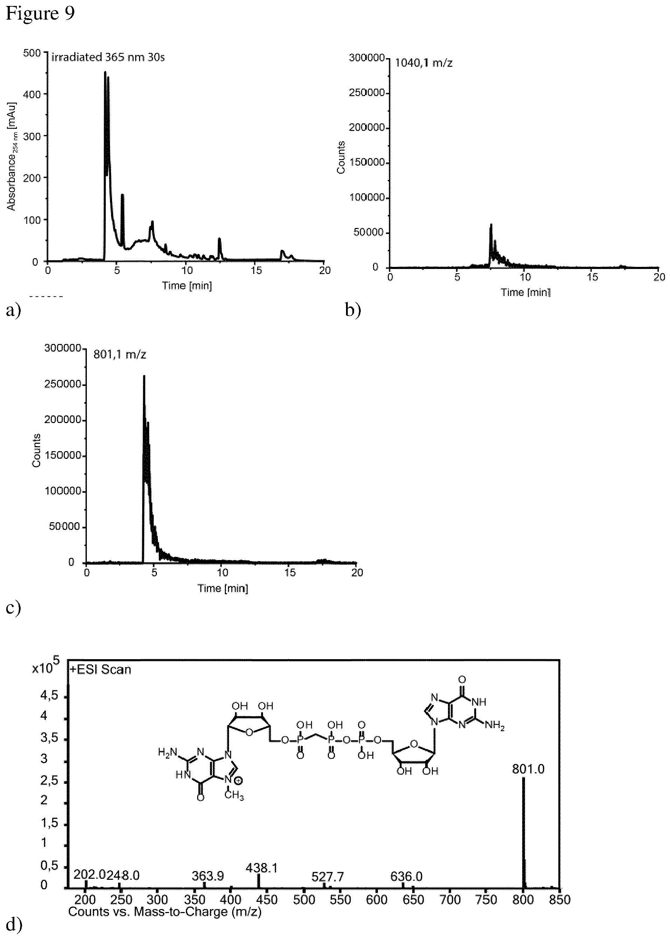

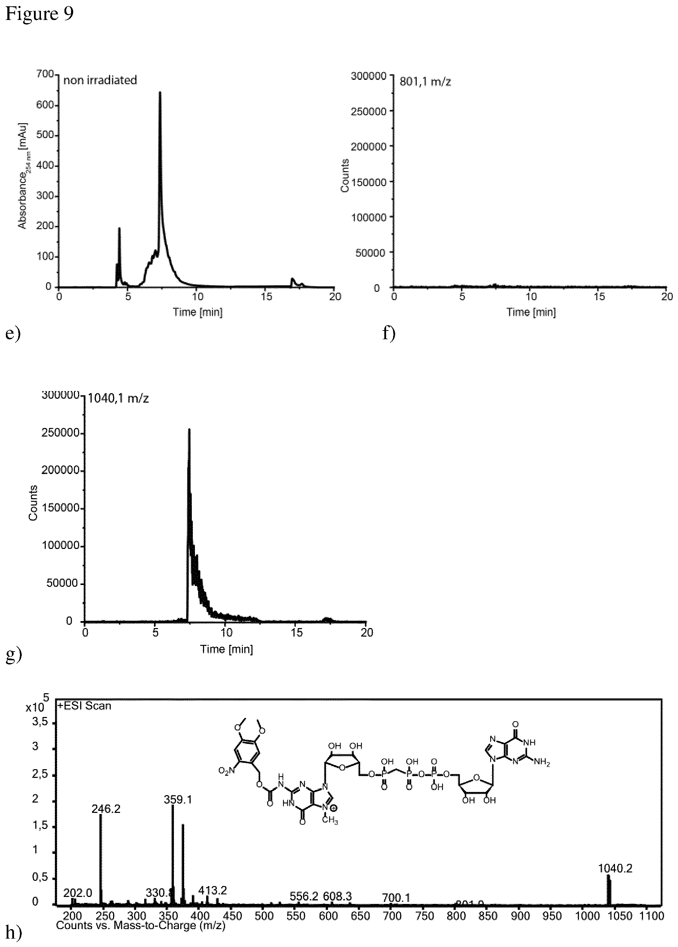

Samples of the photocleavable 5'-cap analog of formula (22) were irradiated for 30 seconds with 365 nm (3 W). The Figure 9 (d and h) shows the LC-MS Data (Triple quad mass measurement).

The Figure 9 shows in Figure 9a) and Figure 9e) the UV-detection channel of the chromatogram showing two main peaks at 4 min and 7.5 min. Figure 9c) shows the Extracted Ion Count (EIC) of 801.1 m/z, which represents the mass of the uncaged m⁷GpCH₂ppG cap analog. This shows that the peak at 4 min results from the uncaging of the cap (calculated mass:[C₂₂H₃₂N₁₀O₁₇P₃]⁺ = 801.1154 [M]⁺; found: 801.1 [M]⁺). The Figure 9b) shows the EIC of 1040.2 m/z (bottom), which represents the DMNB-caged m⁷GpCH₂ppG cap analog. This shows that the peak at 7.5 minutes results from the caged cap (calculated mass: [C₃₂H₄₁N₁₁O₂₃P₃⁺]⁺ = 1040.1584 [M]⁺; found: 1040.2 [M]⁺). This shows that irradiation for 30 seconds with 365 nm results in a sample with partially uncaged cap.

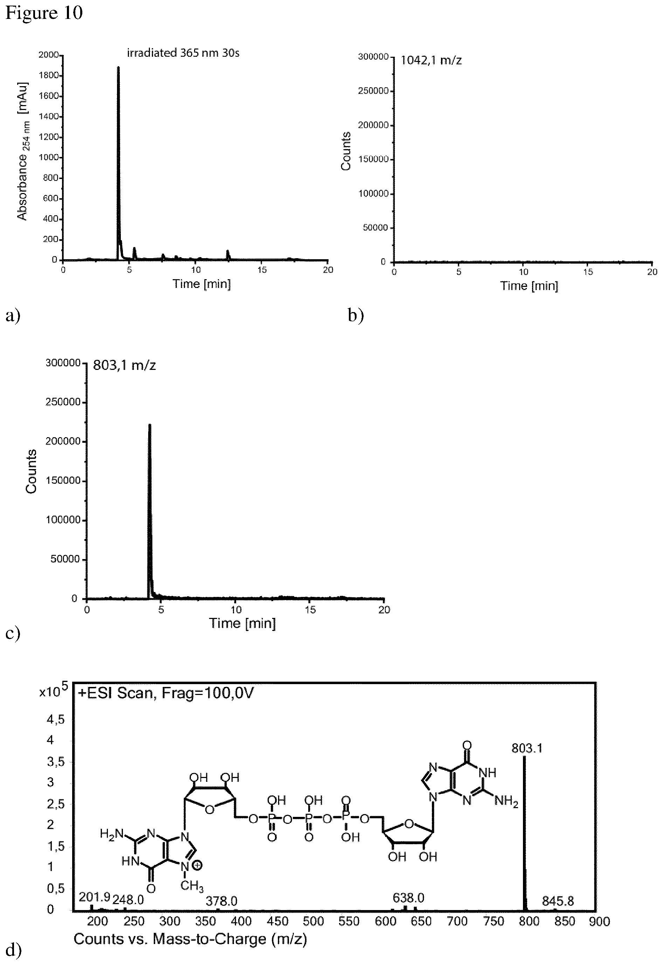

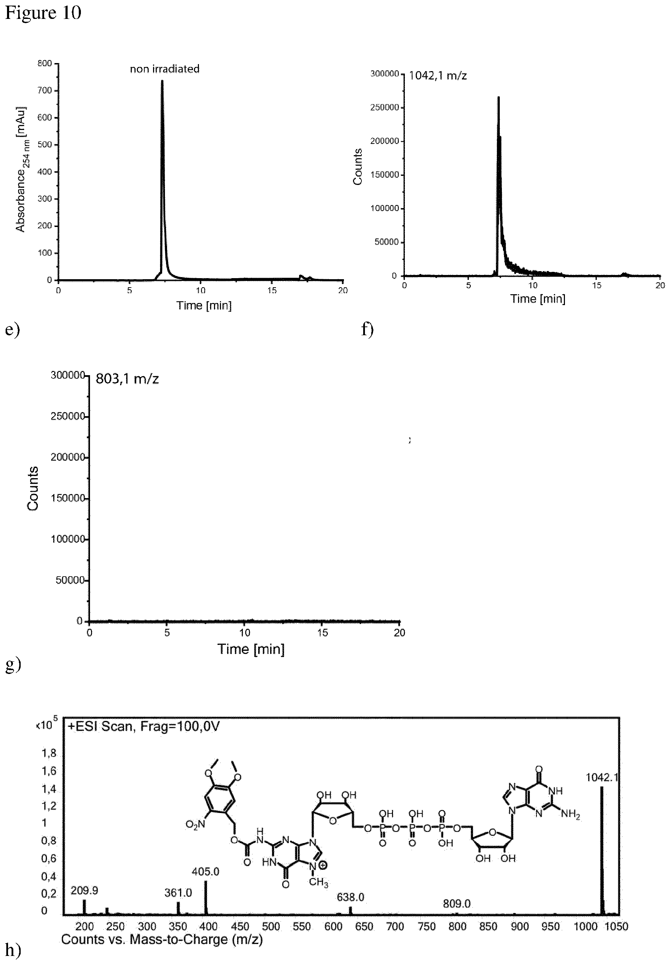

Samples of the photocleavable 5'-cap analog of formula (22) were irradiated for 30 seconds with 365 nm (3 W). The Figure 10 shows the LC-MS Data (Triple quad mass measurement). The Figure 10 shows in Figure 10a) and 10e) the UV-trace of the chromatogram showing two main peaks at 4 min and and 7.5 min). The Extracted Ion Count (EIC) of 803.1 m/z in Figure 10c), which represents the mass of the uncaged m⁷GpppG cap analog shows that the peak at 4 min results from the uncaging of the cap (calculated mass: [C₂₁H₃₀N₁₀O₁₈P₃]⁺ = 803.0947 [M]⁺; found: 803.1 [M]⁺). The EIC of 1042.1 m/z in Figure 10b), which represents the DMNB caged m⁷GpppG cap analog shows that the peak at 7.5 minutes results from the caged cap (calculated mass: [C₃₁H₃₉N₁₁O₂₄P₃]⁺ = 1042.1377 [M]⁺; found: 1042.1 [M]⁺). This shows that irradiation for 30 seconds with 365 nm results in a sample with partially uncaged cap.

### Example 9

### Binding studies of eIF4E with the photocleavable 5'-cap analog of formula (22) and formula (25)

### Expression of eIF4E:

Escherichia coli strain BL21 (DE3) was transformed with the plasmid pET16b-eIF4E and used directly for the inoculation of the 30 mL LB-ampicillin culture. The culture was grown overnight at 37°C with constant shaking at 200 rpm. Five 400 mL LB-ampicillin cultures were each inoculated with 5 mL overnight culture and grown at 37°C, 200 rpm until the OD₆₀₀ reached 0.6. IPTG added to 1 mM and the cells grown further at 37°C, 200 rpm for 3h. The cells were harvested by centrifugation, the cell pellets frozen in liquid nitrogen and stored at 20°C until use.

### Purification of eIF4E:

The thawed cell pellet was resuspended in 10 mL lysis buffer (50 mM sodim phosphate, pH 7.5, 300 mM NaCl, 10 mM imidazole) and PMSF added to 300 µM. The cells were lysed by sonication, 0.5 sec on, 0.5 off at 30% for 3 min (repeated 3 times). The supernatant was obtained by centrifugation at 12,000 rpm for 30 min at 4°C. This was then filtered through 0.4 µM membrane and loaded on 1 ml HisTrap column. The column was washed with 5% buffer B (50 mM sodium phosphate pH 7.5, 300 mM NaCl, 250 mM imidazole) before elution with 5-100% buffer B gradient in 10 c.v. The eluate fractions were pooled and dialyzed against dialysis buffer (20 mM Hepes pH 7.5, 50 mM KCl, 0.2 mM EDTA, 7 mM mercaptoethanol and 0.01% Triton X-100) overnight and then concentrated to 0.3 ml. 10 µl aliquots were frozen in liquid nitrogen and stored at -80°C. The protein concentration was determined by comparison with BSA standard on SDS-PAGE gel.

### Microscale thermophoresis measurements:

Microscale thermophoresis (MST) measurements were performed on a Monolith NT.115 series instrument (NanoTemper). Prior to thermophoresis measurements proteins were labeled by incubation with Cy5-NHS (Lumiprobe) for 30 min at room temperature. Unreacted dye was separated from the protein using PD SpinTrapTM G-25 gel filtration columns (GE Healthcare) according to the manufacturer protocol. Serial dilutions of the cap analogues (starting from 100 µM of cap analog for m7GpppG, 200µM for caged/irradiated caps and 1 mM for the ApppG cap analog) in reaction buffer (20 mM HEPES, 50 mM KCl, 0.2 mM EDTA, Triton-X 0,01% and 7 mM mercaptoethanol) containing 0.025% Tween-20 were prepared and mixed with an equal volume of the labeled protein (-50 nM). The mixture was filled into premium coated capillaries (4 µL) and directly measured. MST power was set to 35%, LED power was set to 20% Red (Exc.: 625nm, Em.: 680 nm). Thermophoresis measurements were performed with the following settings: fluorescence before (5 s), MST on (30 s), fluorescence after (3 s). The capillaries were measured three times in direct succession. MST data were normalized to baseline differences and Kd values were calculated using nonlinear regression assuming a Hill coefficient of 1.0 (GraphPad Prism). Natural cap analog m⁷GpppG was also determined and the ApppG analog was used as negative control.

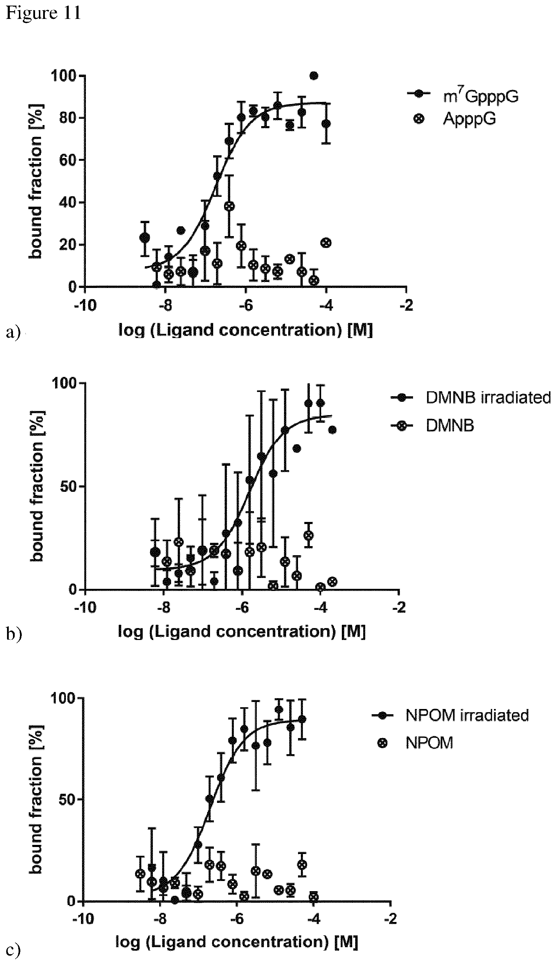

Figure 11 shows the results of binding studies of eIF4E and the natural cap analog m⁷GpppG in Figure 11 a), and the cap analogs of examples 4 (DMNB) and 5 (NPOM) in Figures 11 b) and c) respectively. The cap analog of example 4 and example 5 were tested before and after irradiation ("irradiated"). The indicated Kd values refer to m7GpppG in Figure 11 a), or the measurements after irradiation in Figure 11 b) and c).

### Example 10

### Synthesis of RNA using the photocleavable 5'-cap analogs of formulas (12) and (18)

The photocleavable 5'-cap analogs of formulas (12) and (18) were used for *in vitro* transcription of GLuc mRNA as described above and analysed via agarose gel electrophoresis.

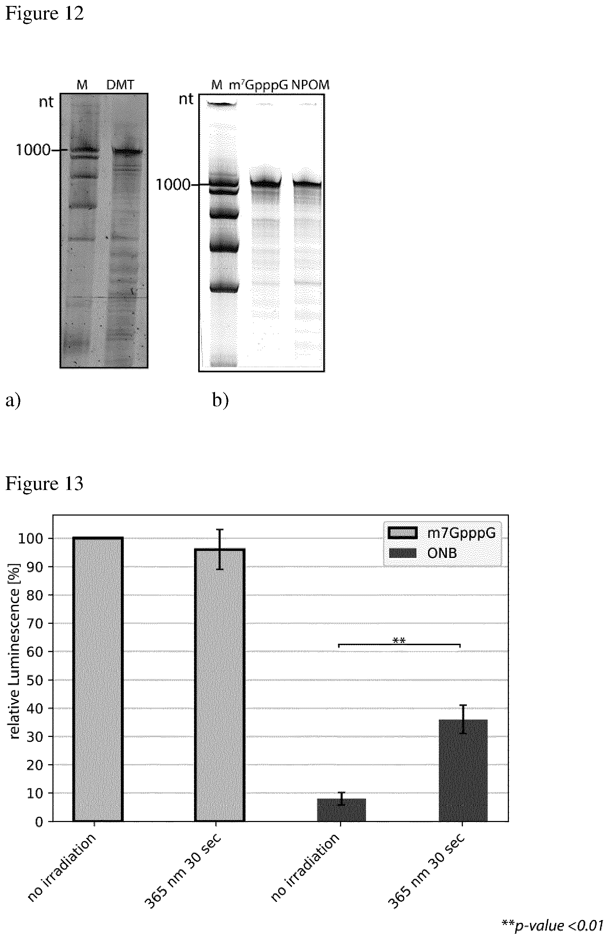

The Figure 12 shows the gel analysis of GLuc mRNA capped with the photocleavable 5'-cap analog of formula (12) denoted "DMT" in Figure 12a) and capped with the photocleavable 5'-cap analog of formula (18) denoted "NPOM" and a commercial non-modified cap analog denoted "m⁷GpppG" (Jena Bioscience) in Figure 12b) using a 7.5 % TBE mini gel (10 W,1h). "M" denotes Riboruler low range RNA ladder. As can be taken from the figure 8, the photocleavable 5'-cap analogs of formulas (12) and (18) was incorporated into the RNA as was the commercial non-modified cap analog used as control.

### Example 11

### Determination of In vitro translation using the photocleavable 5'-cap analog of formula (7)

The photocleavable 5'-cap analog of formula (7) was used for *in vitro* transcription to make FLuc mRNA as described above. This was used for *in vitro* translation and Luminescence measurement as described above. The commercial non-modified cap analog denoted "m⁷GpppG" (Jena Bioscience) was used as positive control. Samples were irradiated for 30 seconds. The ApppG capped mRNA, which is not recognized by the translation initiation factor served as negative control, which is substracted from the other samples as it represents background activity. The experiment was performed in quadruplicates. The FLuc-mRNA capped with the photocleavable 5'-cap analog of formula (7) is denoted "ONB".

Figure 13 shows the results of the *in vitro* translation assay and luminescence measurement with ONB-m⁷GpCH2ppG and m⁷GpppG capped FLuc-mRNA. The values were normalized to the non-irradiated sample with m⁷GpppG capped mRNA (100%). The ApppG capped mRNA, which is not recognized by the translation initiation factor served as negative control, which is substracted from the other samples as it represents background activity. As can be taken from Figure 13, the translation is successfully inhibited to a high degree by the addition of a photocaging group at the N2 position. Upon irradiation the photocaging group is removed and translation is partially restored.

### Example 12

### Determination of in vitro translation using the photocleavable 5'-cap analog of formula (12)

The *in vitro* translation assay and luminescence measurement was performed as described above using the photocleavable 5'-cap analog of formula (12). The capped mRNA was denoted "DMT-m⁷GpCH₂ppG capped FLuc-mRNA". Samples were irradiated for 30 seconds (365 nm) or 60 seconds (405 nm). The commercial non-modified cap analog denoted "m⁷GpppG" (Jena Bioscience) was used as positive control and all other samples were normalized to it. The ApppG capped mRNA, which is not recognized by the translation initiation factor served as negative control, which is substracted from the other samples as it represents background activity. The experiment was performed in triplicates, except *DMT-m7GpCH₂ppG (365 nm) which was a single determination.

It could be seen that the translation was successfully inhibited to a high degree by the addition of a photocaging group at the N2 position. Upon irradiation the photocaging group was removed and translation was partially restored.

### Example 13

### Determination of in vitro translation using the photocleavable 5'-cap analogs of formula (22) and formula (25)

The *in vitro* translation assay in reticulocyte lysate and luminescence measurement was performed as described above using m⁷GpppG capped FLuc-mRNA caged with the photocleavable 5'-cap analog of formulas (22) and (25) (denoted "DMNB m⁷GpppG" and "NPOM m⁷GpppG"). Samples were irradiated for 30 seconds at 365 nm. The ApppG capped mRNA, which is not recognized by the translation initiation factor served as negative control, which was substracted from the other samples as it represents background activity. The values were normalized to the non-irradiated sample with m⁷GpppG capped mRNA (100%). The experiment was performed in triplicates.

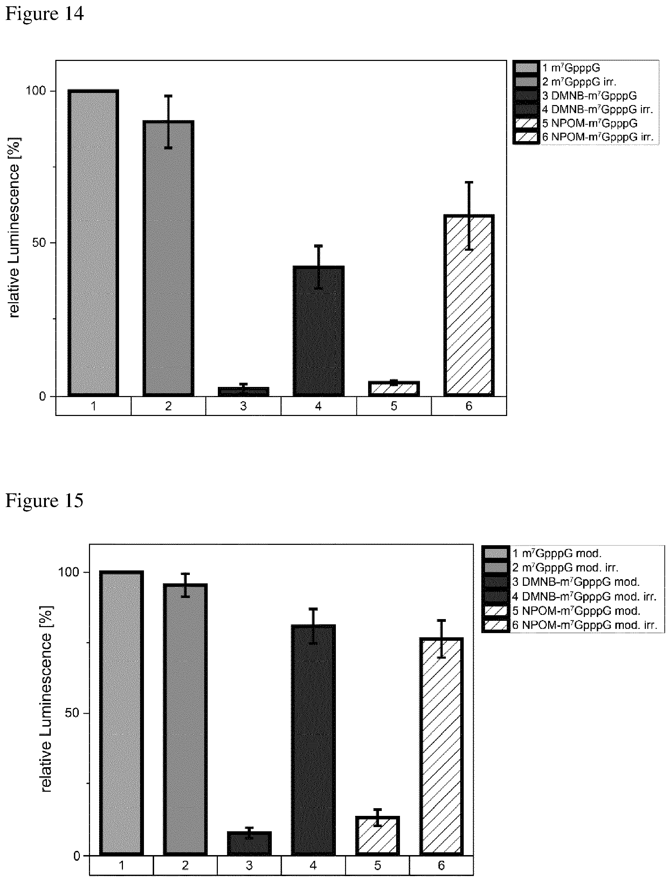

Figure 14 shows the results of the luminescence measurements. As can be taken from Figure 14, a marked increase of translation was achieved by irradiation of the caged mRNA.

### Example 14

### Determination of in vitro translation using modified photocleavable 5'-cap analogs of formula (22) and formula (25)

The *in vitro* translation assay in reticulocyte lysate and luminescence measurement was performed as described above using m⁷GpppG capped FLuc-mRNA with modified nucleotides m⁵C and N¹methyl-pseudouridine caged with the photocleavable 5'-cap analog of formulas (22) and (25) (denoted "DMNB m⁷GpppG mod" and "NPOM m⁷GpppG mod"). Samples were irradiated for 30 seconds at 365 nm. The ApppG capped mRNA, which is not recognized by the translation initiation factor served as negative control, which was substracted from the other samples as it represents background activity. The values were normalized to the non-irradiated sample with m⁷GpppG capped mRNA (100%). The experiment was performed in triplicates.

Figure 15 shows the results of the luminescence measurements. As can be taken from Figure 15, a marked increase of translation was achieved by irradiation of the caged mRNA

### Example 15

### Determination of in cell translation upon irradiation of RNA modified with the photocleavable 5'-cap analog of formula (12) for 30 seconds.

In cell translation and irradiation in cell culture of mammalian HeLa cells was performed as described above. GLuc mRNA capped with the photocleavable 5'-cap analog of formula (12) ("DMT") was used and the commercial non-modified cap analog denoted "m⁷GpppG" (Jena Bioscience) was used as a control. HeLa cells were either transfected with the DMT-m7GpCH₂ppG capped mRNA (irradiated and non-irradiated before transfection) or with the natural capped mRNA (irradiated and non-irradiated before transfection). Irradiation of the mRNA containing the natural cap was performed (5 V, 600 mA) with 365 nm for 30 seconds to evaluate the resulting influence on translational efficiency. DMT-m⁷GpCH₂ppG capped mRNA was irradiated in the same way or with 405 nm for 60 seconds. The ApppG capped mRNA, which is not recognized by the translation initiation factor served as negative control, which is substracted from the other samples as it represents background activity. The values were normalized to the non-irradiated m⁷GpppG capped sample (100%). The experiment was performed in triplicates.

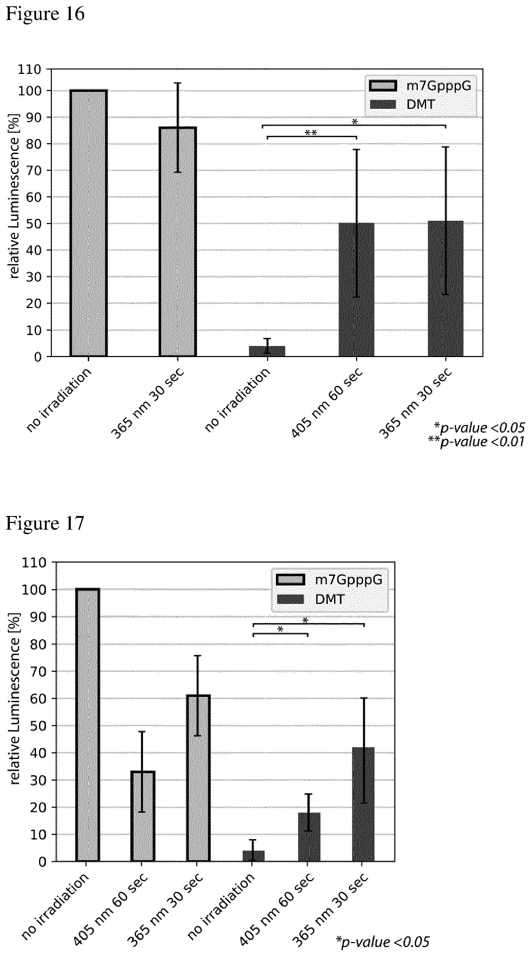

The Figure 16 shows the results of the luminescence measurement of cell culture media after translation in cells transfected with GLuc mRNA. As can be taken from Figure 16, a significant increase of translation was achieved by irradiation of the caged mRNAs.

### Example 16

### Determination of in-cell translation efficiency upon irradiation of RNA modified with the photocleavable 5'-cap analog of formula (12) for 30 and 60 seconds

The luminescence measurement of cell culture media after translation in cells transfected with modified and non-modified GLuc mRNA was repeated using irradiation with 365 nm/405 nm for different periods of time. HeLa cells were either transfected with the GLuc mRNA capped with the photocleavable 5'-cap analog of formula (12) denoted "DMT-m⁷GpCH₂ppG capped mRNA" or with the natural m⁷GpppG capped mRNA. Cells transfected with the natural capped mRNA were irradiated (5 V, 600 mA) with 365 nm/405 nm for 30 s or 60 s to evaluate the resulting influence on translational efficiency. Cells transfected with DMT-m7GpCH₂ppG capped mRNA were irradiated in the same way. The ApppG capped mRNA, which is not recognized by the translation initiation factor served as negative control, which is substracted from the other samples as it represents background activity. The values were normalized to the non-irradiated cells with m⁷GpppG capped mRNA (100%). The experiment was performed in triplicates.

Figure 17 shows translation in cells as luminescence measurement after irradiation of HeLa cells transfected with GLuc-mRNA capped with the photocleavable 5'-cap analog of example 3 (DMT) and a commercial non-modified cap analog (m⁷GpppG) with 365 nm/405 nm for 30 s or 60 s. As can be taken from Figure 17, again a significant increase of translation was achieved by irradiation with either irradiation at 365 nm and 405 nm for 30 s or 60 s.

### Example 17

### Determination of in-cell translation efficiency upon irradiation of RNA modified with the photocleavable 5'-cap analog of formula (12) for 15, 25 and 30 seconds

The luminescence measurement as described in example 16 was repeated using shorter periods of irradiation of 15, 25 and 30 seconds.

Luminescence measurement of cell culture media after translation of transfected modified and non-modified GLuc mRNA in cells. HeLa cells were either transfected with the NPOM-m7GpCH₂ppG capped mRNA or with the natural m⁷GpppG capped mRNA. Cells transfected with the natural capped mRNA were irradiated (5 V, 600 mA) with 365 nm/405 nm for different periods of time to evaluate the resulting influence on translational efficiency. Cells transfected with NPOM-m⁷GpCH₂ppG capped mRNA were irradiated in the same way. The ApppG capped mRNA, which is not recognized by the translation initiation factor served as negative control, which is substracted from the other samples as it represents background activity. The values were normalized to the non-irradiated cells with m⁷GpppG capped mRNA (100%). The experiment was performed in triplicates.

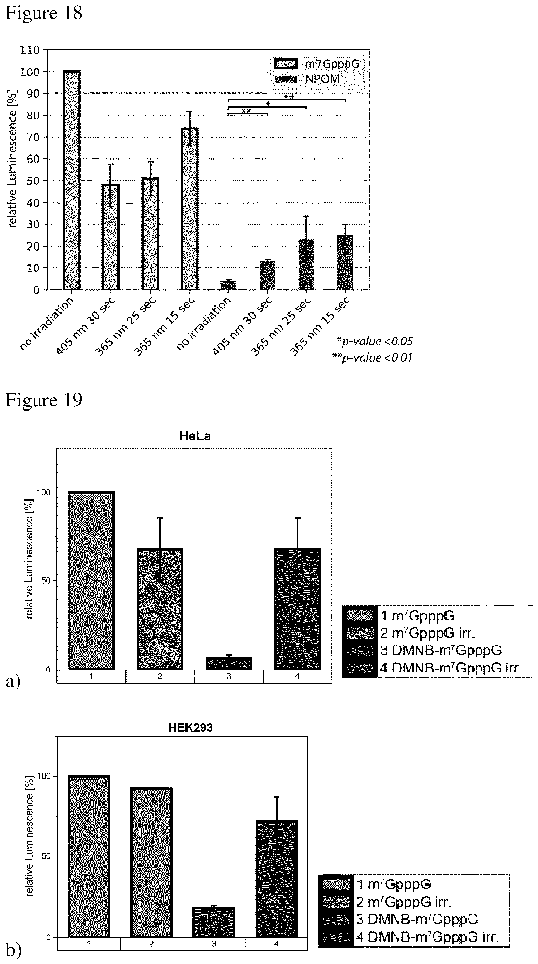

Figure 18 shows the results of the luminescence measurement of cells transfected with and translating GLuc mRNA after the cells were irradiated with 365 nm or 405 nm for 15 s, 25 s or 30 s. As can be taken from Figure 18, again a significant increase of translation was achieved by irradiation with shorter wavelengths (365 nm).

### Example 18

### In cell translation assays using the photocleavable 5'-cap analog of formula (22)

In cell translation assay in HeLa and HEK293T cells with m⁷GpppG capped GLuc-mRNA caged with the photocleavable 5'-cap analog of formula (22) (denoted "DMNB m⁷GpppG") with either A) regular nucleotides or B) modified nucleotides m⁵C and N¹methyl-pseudouridine. Samples were irradiated for 30 seconds (365 nm). The ApppG capped mRNA, which is not recognized by the translation initiation factor served as negative control, which is substracted from the other samples as it represents background activity. The values were normalized to the non-irradiated sample with m⁷GpppG capped mRNA (100%). The experiment was performed in triplicates.

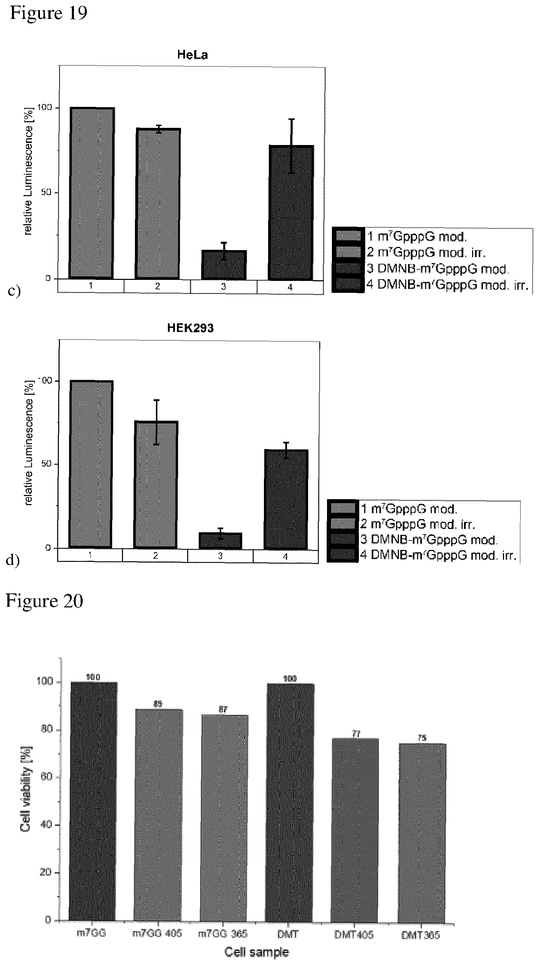

Figure 19 shows the results of the luminescence measurement of cells transfected with and translating GLuc mRNA. As can be taken from Figure 19, a marked increase of translation was achieved by irradiation of the cells.

### Example 19

### Determination of cell viability

To evaluate the cell viability after irradiation, a MTT test was used as described above. HeLa cells were irradiated (5 V, 600 mA) with either 365 nm (m7GG 365, DMT 365) or 405 nm (m7GG 405, DMT 405) for 30 seconds (365 nm) or 60 seconds (405 nm). The values were normalized to the non-irradiated sample (m7GG).

Figure 20 shows the cell viability after irradiation determined via the MTT test. The results are based on a single determination. As can be taken from Figure 20, cell viability was only slightly decreased. This shows that neither the use of the photocleavable 5'-cap analogs nor the irradiation used for cleavage is toxic.

### Example 20

### Determination of translation efficiency of cell supernatant

The translation efficiency was determined by luminescence measurement of the supernatant of HeLa cells, which were transfected with differently capped GLuc mRNAs. The control cells were not transfected with mRNA. The cells transfected with ARCA and ONB capped mRNAs were each irradiated (5 V, 200 mA, 365 nm) for 45 s. The values were normalized to the value of the sample transfected with mRNA capped with mRNA capped with m⁷GpppG (m⁷GG). The results are based on a single determination.

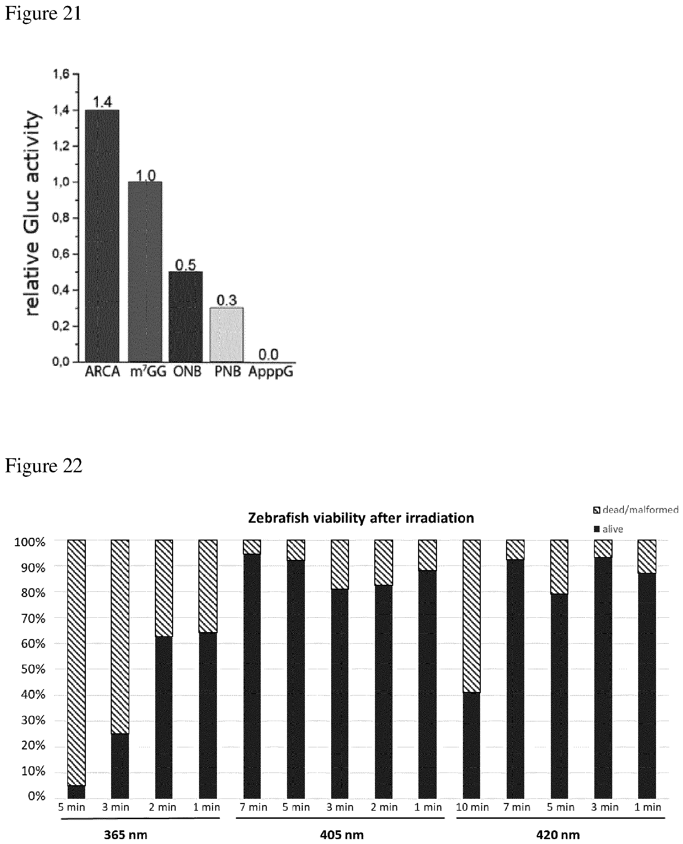

Figure 21 shows the results of the luminescence measurement. As can be taken from Figure 21, an addition of a substituent like ONB (ortho-nitrobenzyl) or PNB (para-nitrobenzyl) at the N2 position impacts the translation efficiency in an inhibiting way.

### Example 21

### Determination of effect of irradiation on viability in vivo in Zebrafish

### Zebrafish Husbandry:

All experiments were performed in accordance with internationally recognized guidelines for the use of fish in biomedical research. Embryos were raised at 28 °C in E3 medium and staged in hours post fertilization (hpf) and by morphological criteria.

### Zebrafish irradiation:

30 Zebrafish embryos were transferred in a 96 well plate well, filled up with E3 buffer and subsequently irradiated within the first hpf with either 365 nm, 405 nm or 420 nm for different periods of time of 1, 2, 3, 5, 7 or 10 min. The development and viability of the embryos was supervised at 6 hpf, 19 hpf, 25 hpf and 30 hpf by microscopy (Nikon SMZ1270). Normal developed fish embryos were counted as "alive". Dead fish embryos and strong malformation of the fish embryos was counted as "dead". Dead and malformed embryos were counted and removed at each timepoint.

Figure 22 shows the percentage of dead embryos and alive zebrafish embryos after irradiation with 365 nm, 405 nm or 420 nm for 1, 2, 3, 5, 7 or 10 min, where the black bars represent the viable fraction. As can be taken from the mortality rates of Figure 22, the irradiation as used was compatible with the Zebrafish embryos.

In summary the results show that photocleavable 5'-cap analogs are provided that allow a photocontrollable regeneration of the native cap upon radiation *in vitro* and *in vivo.*

## Claims

1. A photocleavable 5'-cap analog according to formula (I) as given as follows or salts thereof, wherein the 5'-cap analog comprises a photocleavable group A fused via a carbamate moiety to the 5'-cap structure: 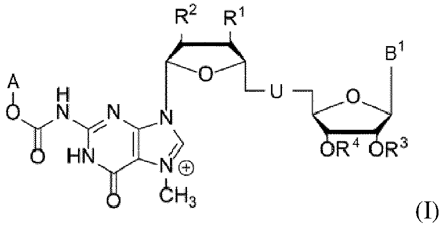 wherein:
R¹, R² are independently H, OH or OCH₃;
R³ is H or CH₃;
R⁴ is H or an element according to formula (II) 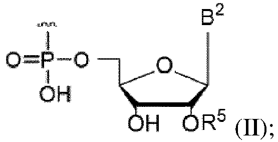 wherein R⁵ is H or CH₃;
U is a modified or unmodified oligophosphate or methyleneoligophosphate group;
B¹, B² is a modified or unmodified nucleobase; and
A is a photocleavable group selected from the group of ortho-nitrobenzyl-based groups, o-nitro-2-phenethyl-based groups, (coumarin-4-yl)methyl-based groups, benzocoumarin-based groups, 2-thionated coumarin-based groups, arylmethyl and arylcarbonylmethyl-based groups, xanthene-based groups, 1,4-benzoquinone-based groups, bimane-based groups, 10H-phenothiazine-based groups, and BODIPY-based groups.

2. The photocleavable 5'-cap analog according to claim 1, wherein A is a photocleavable group selected from the group of ortho-nitrobenzyl-based groups according to formulas (A1) to (A5), o-nitro-2-phenethyl-based groups according to formulas (A6) and (A7), (coumarin-4-yl)methyl-based groups according to formulas (A8) and (A11) to (A14), 2-thionated coumarin-based groups according to formulas (A9) and (A10), benzocoumarin-based groups according to formulas (A15) and (A16), arylmethyl and arylcarbonylmethyl-based groups according to formulas (A17) to (A20), xanthene-based groups according to formula (A21), 1,4-benzoquinone-based groups according to formulas (A22) to (A25), bimane-based groups according to formula (A26), 10H-phenothiazine-based groups according to formula (A27), and BODIPY-based groups according to formulas (A28) to (A34) as given as follows: 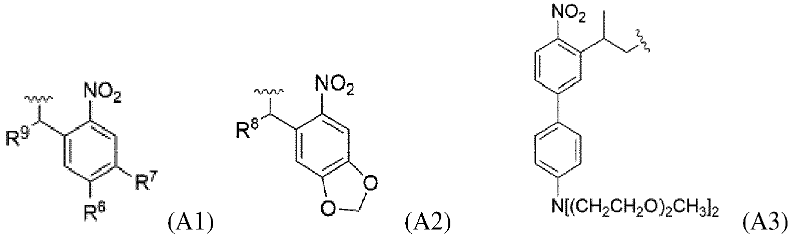 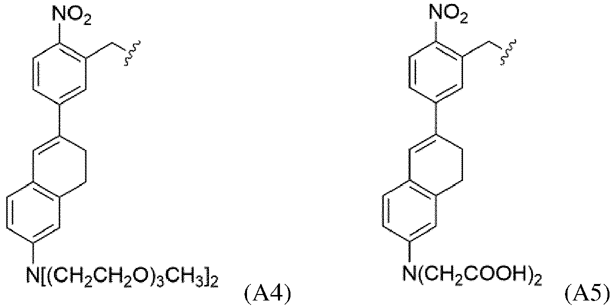 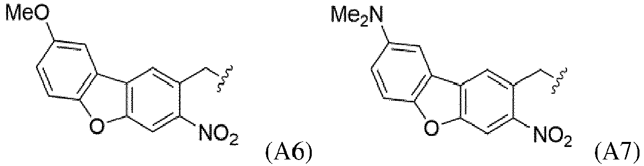 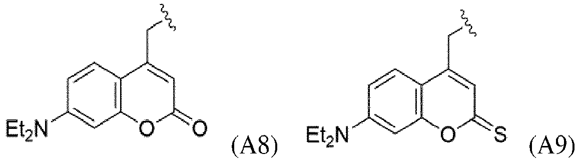 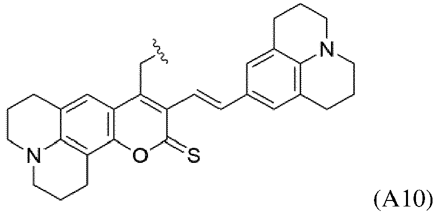 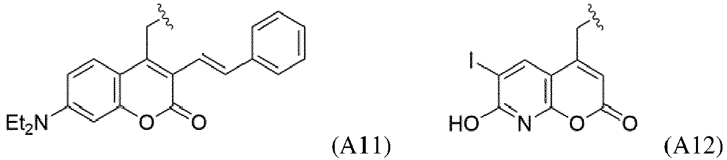 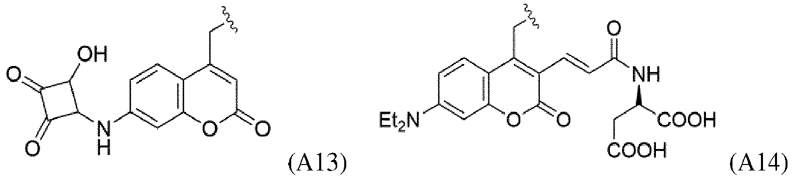 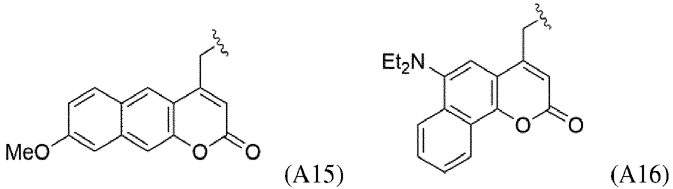 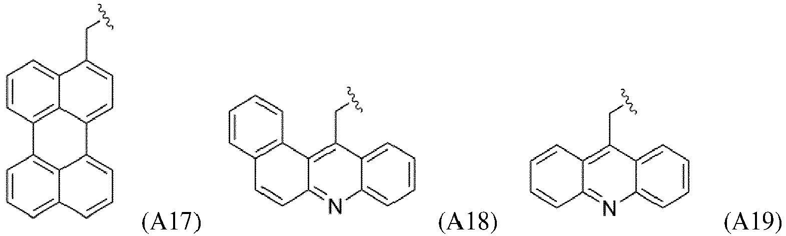 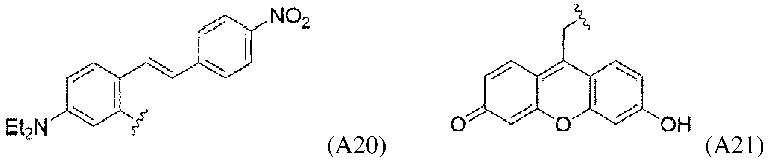 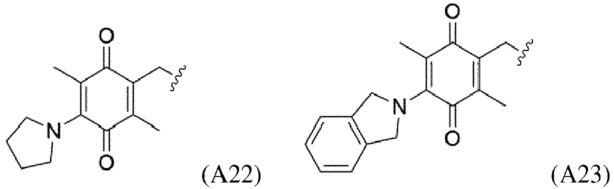 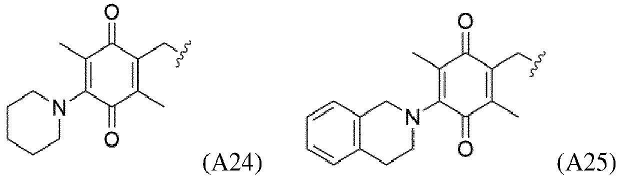 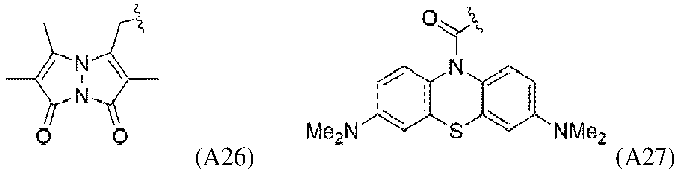 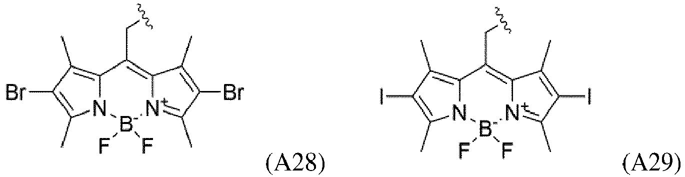 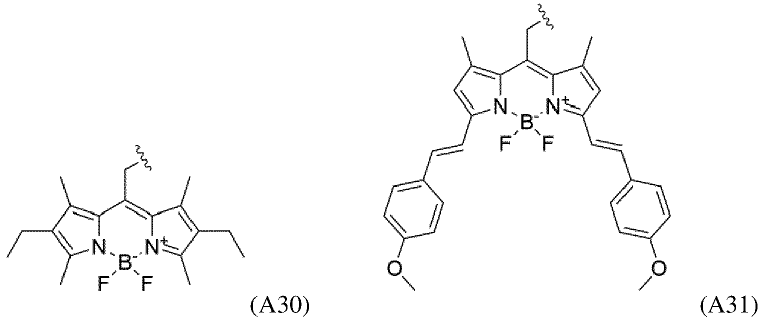 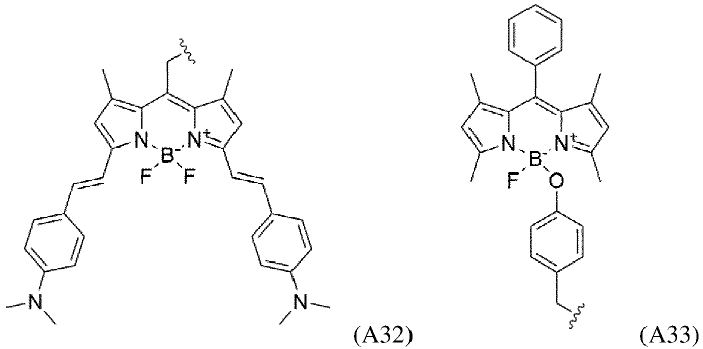 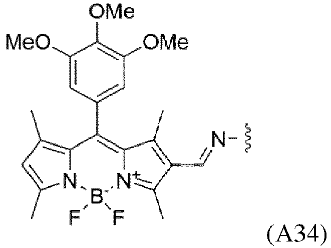 wherein:
R⁶, R⁷ are independently selected from the group of H, OH, NH2, NH2, -NH(C₁-C₁₀), -N(C₁-C₁₀)₂, N-methyl piperazine, Br, Cl, C₁-C₁₀ aliphatic alkoxy or benzyloxy;
R⁸, R⁹ is H or CH₃;
Me is methyl; and
Et is ethyl.

3. The photocleavable 5'-cap analog according to claim 1 or 2, wherein A is a photocleavable group selected from the group of ortho-nitrobenzyl compounds according to formulas (A40) to (A44) as given as follows:

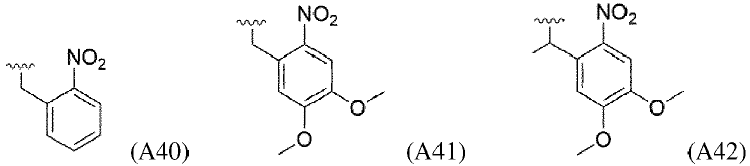

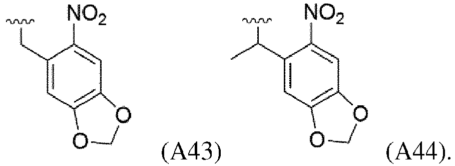

4. The photocleavable 5'-cap analog according to any one of the foregoing claims, wherein B¹ and B² independently are a natural nucleobase selected from guanine or adenine.

5. The photocleavable 5'-cap analog according to any one of the foregoing claims, wherein U is a oligophosphate or methyleneoligophosphate group selected from the group of compounds according to formulas (U1) to (U4) as given as follows or salts thereof: 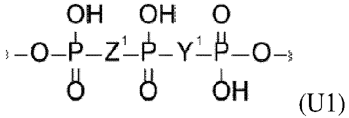 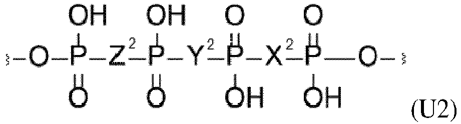 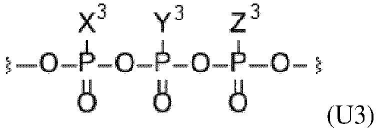 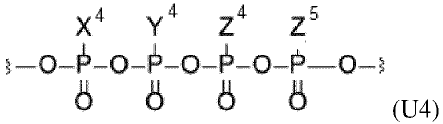 wherein:
Y¹ , Z¹ are independently selected from the group of O, NH and CH2;
X², Y², Z² are independently selected from the group of O, NH and CH2;
X³, Y³, Z³ are independently selected from the group of O, S, Se and BH3;
X⁴, Y⁴, Z⁴, Z⁵ are independently O or S.

6. The photocleavable 5'-cap analog according to any one of the foregoing claims, wherein U is a modified oligophosphate or methyleneoligophosphate group selected from the group of compounds according to formulas (U5) to (U15) as given as follows or salts thereof:

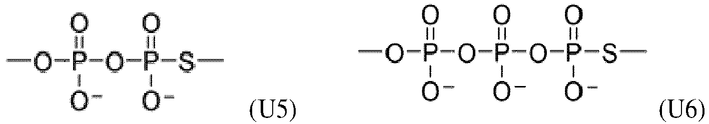

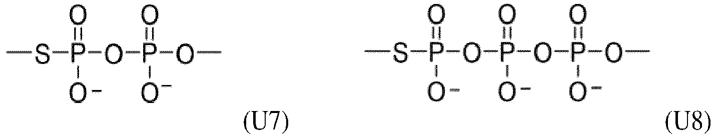

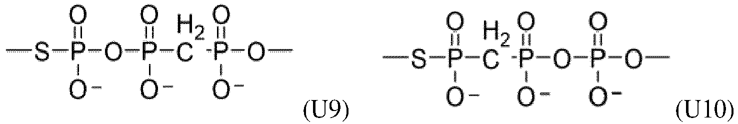

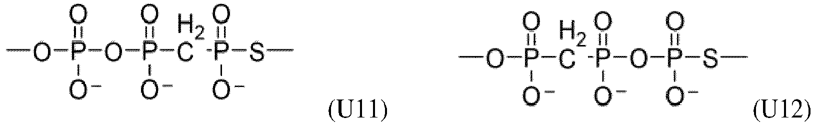

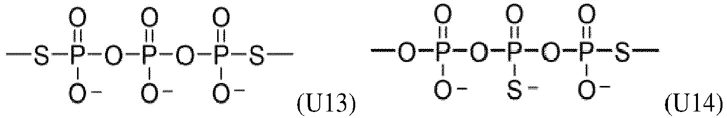

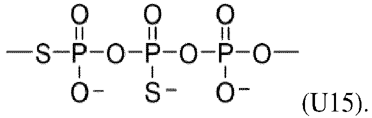

7. The photocleavable 5'-cap analog according to any one of claims 1 to 6, wherein the photocleavable 5'-cap analog has a structure according to formula (7) as given as follows or salts thereof:

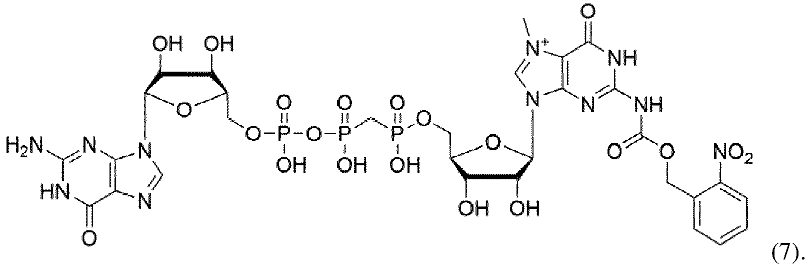

8. The photocleavable 5'-cap analog according to any one of claims 1 to 6, wherein the photocleavable 5'-cap analog has a structure according to formula (12), formula (18), formula (22) or formula (25) as given as follows or salts thereof:

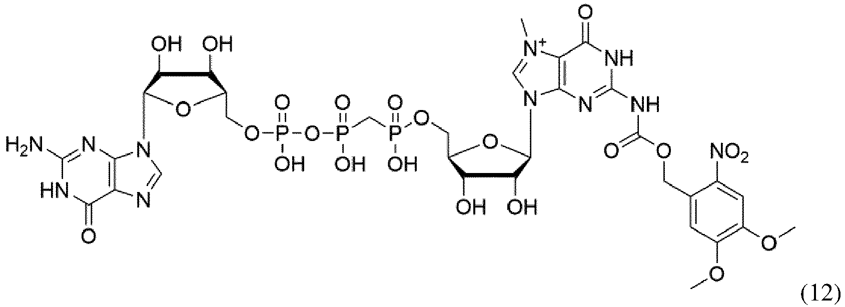

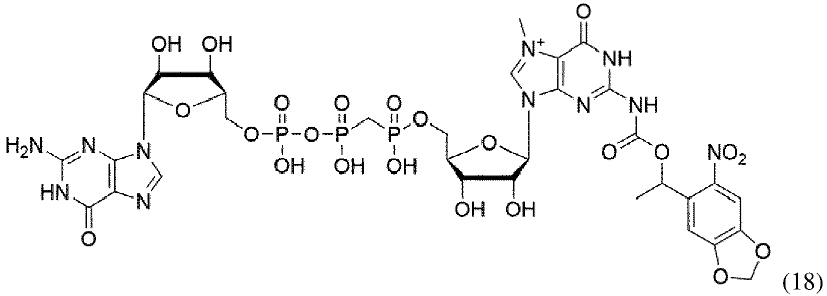

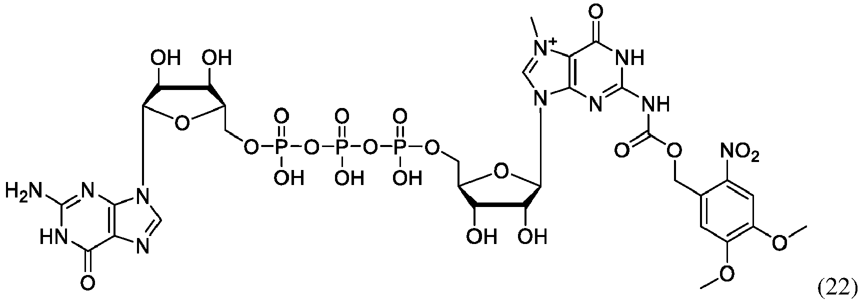

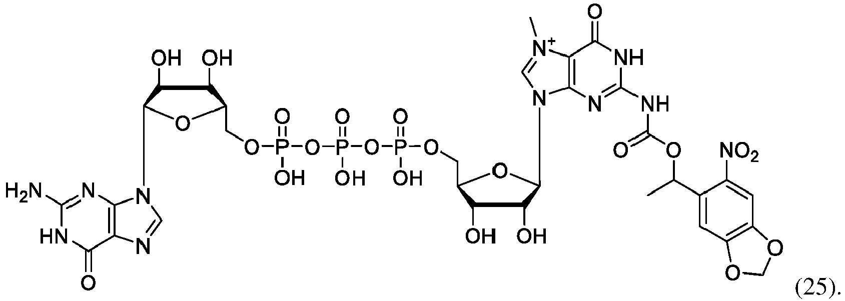

9. The photocleavable 5'-cap analog according to any one of claims 1 to 8 for use as a medicament, particularly for use in the treatment of pain or cancer.

10. An RNA molecule comprising a photocleavable 5'-cap analog according to any one of claims 1 to 8.

11. The RNA molecule according to claim 10 for use as a medicament, particularly for use as a vaccine or for use in protein replacement therapy or for use in the treatment of pain or cancer.

12. A pharmaceutical composition comprising a photocleavable 5'-cap analog according to any one of claims 1 to 8 or an RNA molecule comprising a photocleavable 5'-cap analog according to any one of claims 1 to 8 and a pharmaceutically acceptable carrier.

13. Use of a photocleavable 5'-cap analog according to any one of claims claim 1 to 8 in a method of synthesising an RNA molecule, or for inhibiting cap-binding proteins, such as cap-degrading enzymes.

14. A method for synthesising an adenosine or guanosine fused via a carbamate moiety to an o-nitrobenzyl group, the method comprising the steps of:
a) providing a nucleoside selected from adenosine or guanosine;
b) reacting the hydroxyl and carbonyl oxygens of the nucleoside with trimethylsilyl chloride to form a protected nucleoside by trimethylsilyl group;
c) reacting the amino group of adenosine or guanosine with phosgene to an isocyanate group;
d) reacting the isocyanate group with optionally substituted o-nitrobenzyl alcohol or an optionally substituted compound according to formula (6) as follows: 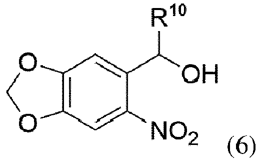 wherein R¹⁰ is selected from H or methyl
to yield the nucleoside fused to o-nitrobenzyl, 4,5-dimethoxy-2-nitrobenzyl or the optionally substituted compound according to formula (6) via a carbamate moiety; and
e) optionally removing the trimethylsilyl groups.

15. A method for synthesising a photocleavable 5'-cap analog comprising a carbamate moiety according to any one of claims 1 to 8, the method comprising synthesising an adenosine or guanosine fused via a carbamate moiety to an o-nitrobenzyl group according to claiml4.
